(19) Europäisches Patentamt / European Patent Office / Office européen des brevets

(11) **EP 2 216 042 A1**

(12) # EUROPEAN PATENT APPLICATION

(43) Date of publication:
**11.08.2010 Bulletin 2010/32**

(51) Int Cl.:
*A61K 38/26* (2006.01)     *A61K 9/08* (2006.01)

(21) Application number: **09290090.1**

(22) Date of filing: **09.02.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **IPSEN PHARMA S.A.S.**
**91200 Boulogne-Billancourt (FR)**

(72) Inventors:
• Mondoly, Nathalie
**78150 Le Chesnay (FR)**
• Brochard, Anne
**28100 Dreux (FR)**

(74) Representative: **Bourgouin, André et al**
**Ipsen Pharma S.A.S.**
**Direction de la Propriété Intellectuelle**
**65 quai Georges Gorse**
**91200 Boulogne Billancourt (FR)**

(54) **GLP-1 analogues pharmaceutical compositions**

(57)     The present invention is directed to sustained release liquid pharmaceutical compositions comprising a liquid, a peptide analogue according to the formula [Aib$^{8,35}$]hGLP-1(7-36)NH$_2$, a divalent metal and/or divalent metal salt, and an acetate salt and/or acetic acid. The invention also relates to containers comprising the pharmaceutical compositions and methods for preparing the pharmaceutical compositions.

EP 2 216 042 A1

**Description**

Background of the Invention

The present invention relates to improvements in compositions containing peptide analogues of glucagon-like peptide-1 and/or pharmaceutically-acceptable salts thereof, pharmaceutical compositions, methods for preparing such compositions and uses thereof.

Glucagon-like peptide-1 (7-36) amide (GLP-1) is synthesized in the intestinal L-cells by tissue-specific post-translational processing of the glucagon precursor preproglucagon (Varndell, J.M., et al., J. Histochem Cytochem, 1985: 33:1080-6) and is released into the circulation system in response to a meal. The plasma concentration of GLP-1 rises from a fasting level of approximately 15 pmol/L to a peak postprandial level of 40 pmol/L. It has been demonstrated that, for a given rise in plasma glucose concentration, the increase in plasma insulin is approximately threefold greater when glucose is administered orally compared with intravenously (Kreymann, B., et al., Lancet 1987:2, 1300-4). This alimentary enhancement of insulin release, known as the incretin effect, is primarily humoral and GLP-1 is thought to be the most potent physiological incretin in humans. In addition to the insulinotropic effect, GLP-1 suppresses glucagon secretion, delays gastric emptying (Wettergren A., et al., Dig Dis Sci 1993:38:665-73) and may enhance peripheral glucose disposal (D'Alessio, D.A. et al., J. Clin Invest 1994:93:2293-6).

In 1994, the therapeutic potential of GLP-1 was suggested following the observation that a single subcutaneous (s/c) dose of GLP-1 could completely normalize postprandial glucose levels in patients with non-insulin-dependent diabetes mellitus (NIDDM) (Gutniak, M.K., et al., Diabetes Care 1994:17:1039-44). This effect was thought to be mediated both by increased insulin release and by a reduction in glucagon secretion. Furthermore, an intravenous infusion of GLP-1 has been shown to delay postprandial gastric emptying in patients with NIDDM (Williams, B., et al., J. Clin Endo Metab 1996:81:327-32). Unlike sulphonylureas, the insulinotropic action of GLP-1 is dependent on plasma glucose concentration (Holz, G.G. 4th, et al., Nature 1993:361:362-5). Thus, the loss of GLP-1-mediated insulin release at low plasma glucose concentration protects against severe hypoglycemia. This combination of actions gives GLP-1 unique potential therapeutic advantages over other agents currently used to treat NIDDM.

Numerous studies have shown that when given to healthy subjects, GLP-1 potently influences glycemic levels as well as insulin and glucagon concentrations (Orskov, C, Diabetologia 35:701-711, 1992; Holst, J.J., et al., Potential of GLP-1 in diabetes management in Glucagon III, Handbook of Experimental Pharmacology, Lefevbre PJ, Ed. Berlin, Springer Verlag, 1996, p. 311-326), effects which are glucose dependent (Kreymann, B., et al., Lancet ii: 1300-1304, 1987; Weir, G.C., et al., Diabetes 38:338-342, 1989). Moreover, it is also effective in patients with diabetes (Gutniak, M., N. Engl J Med 226:1316-1322, 1992; Nathan, D.M., et al., Diabetes Care 15:270-276, 1992), normalizing blood glucose levels in type 2 diabetic subjects (Nauck, M.A., et al., Diabetologia 36:741-744, 1993), and improving glycemic control in type 1 patients (Creutzfeldt, W.O., et al., Diabetes Care 19:580-586, 1996), demonstrating its ability to, *inter alia*, increase insulin sensitivity/reduce insulin resistance. GLP-1 and agonists thereof have been proposed for use in subjects at risk for developing non-insulin dependent diabetes (see WO 00/07617) as well as for the treatment of gestational diabetes mellitus (U.S. Patent Pub. No. 20040266670).

In addition to the foregoing, there are a number of therapeutic uses in mammals, e.g., humans, for which GLP-1 and agonists thereof have been suggested, including, without limitation: improving learning, enhancing neuro-protection, and/or alleviating a symptom of a disease or disorder of the central nervous system, e.g., through modulation of neurogenesis, and e.g., Parkinson's Disease, Alzheimer's Disease, Huntington's Disease, ALS, stroke, ADD, and neuropsychiatric syndromes (U.S. Patent Pub. No.'s 20050009742 and 20020115605); converting liver stem/progenitor cells into functional cells pancreatic (WO03/033697); preventing beta-cell deterioration (U.S. Patent Pub. No.'s 20040053819 and 20030220251) and stimulation of beta-cell proliferation (U.S. Patent Pub. No. 20030224983); treating obesity (U.S. Patent Pub. No. 20040018975; WO98/19698); suppressing appetite and inducing satiety (U.S. Patent Pub. No. 20030232754); treating irritable bowel syndrome (WO 99/64060); reducing the morbidity and/or mortality associated with myocardial infarction (US Patent Pub No 20040162241, WO98/08531) and stroke (see WO 00/16797); treating acute coronary syndrome characterized by an absence of Q-wave myocardial infarction (U.S. Patent Pub. No. 20040002454); attenuating post-surgical catabolic changes (US Patent No. 6,006,753); treating hibernating myocardium or diabetic cardiomyopathy (U.S. Patent Pub. No. 20050096276); suppressing plasma blood levels of norepinepherine (U.S. Patent Pub. No. 20050096276); increasing urinary sodium excretion, decreasing urinary potassium concentration (U.S. Patent Pub. No. 20050037958); treating conditions or disorders associated with toxic hypervolemia, e.g., renal failure, congestive heart failure, nephrotic syndrome, cirrhosis, pulmonary edema, and hypertension (U.S. Patent Pub. No. 20050037958); inducing an inotropic response and increasing cardiac contractility (U.S. Patent Pub. No. 20050037958); treating polycystic ovary syndrome (U.S. Patent Pub. No.'s 20040266678 & 20040029784); treating respiratory distress (U.S. Patent Pub. No. 20040235726); improving nutrition via a non-alimentary route, i.e., via intravenous, subcutaneous, intramuscular, peritoneal, or other injection or infusion (U.S. Patent Pub. No. 20040209814); treating nephropathy (U.S. Patent Pub. No. 20040209803); treating left ventricular systolic dysfunction, e.g., with abnormal left ventricular ejection fraction (U.S. Patent Pub. No. 20040097411); inhibiting antro-duodenal motility, e.g., for the treatment or prevention of gastrointestinal disorders such as diarrhea, postoperative dumping syndrome and irritable bowel syndrome, and as premedication in endoscopic procedures (U.S. Patent Pub. No. 20030216292); treating critical illness polyneuropathy (CIPN) and systemic inflammatory response syndrome (SIRS) (U.S. Patent Pub. No. 20030199445); modulating triglyceride levels and treating dyslipidemia (U.S. Patent Pub. No.'s 20030036504 and 20030143183); treating organ tissue injury caused by reperfusion of blood flow following ischemia (U.S. Patent Pub. No. 20020147131); treating coronary heart disease risk factor (CHDRF) syndrome (U.S. Patent Pub. No. 20020045636); and others.

GLP-1 is, however, metabolically unstable, having a plasma half-life ($t_{1/2}$) of only 1-2 min *in vivo.* Exogenously administered GLP-1 is also rapidly degraded (Deacon, C.F., et al., Diabetes 44:1126-1131, 1995). This metabolic instability limits the therapeutic potential of native GLP-1. A number of attempts have been taken to improve the therapeutic potential of GLP-1 and its analogs through improvements in formulation. For example, International patent publication no. WO 01/57084 describes a process for producing crystals of GLP-1 analogues which are said to be useful in the preparation of pharmaceutical compositions, such as injectable drugs, comprising the crystals and a pharmaceutical acceptable carrier. Heterogeneous micro crystalline clusters of GLP-1 (7-37)OH have been grown from saline solutions and examined after crystal soaking treatment with zinc and/or m-cresol (Kim and Haren, Pharma. Res. Vol. 12 No. 11 (1995)). Crude crystalline suspensions of GLP(7-36)$NH_2$ containing needle-like crystals and amorphous precipitation have been prepared from phosphate solutions containing zinc or protamine (Pridal, et. al., International Journal of Pharmaceutics Vol. 136, pp. 53-59 (1996)). European patent publication no. EP 0619322A2 describes the preparation of micro-crystalline forms of GLP-1 (7-37)OH by mixing solutions of the protein in pH 7-8.5 buffer with certain combinations of salts and low molecular weight polyethylene glycols (PEG). U.S. Patent No. 6,566,490 describes seeding microcrystals of, *inter alia*, GLP-1 which are said to aid in the production of purified peptide products. U.S. Patent 6,555,521 (US '521) discloses GLP-1 crystals having a tetragonal flat rod or a plate-like shape which are said to have improved purity and to exhibit extended in vivo activity. US '521 teaches that such crystals are relatively uniform and remain in suspension for a longer period of time than prior crystalline clusters and amorphous crystalline suspensions which were said to settle rapidly, aggregate or clump together, clog syringe needles and generally exacerbate unpredictable dosing.

A biodegradable triblock copolymer of poly [(dl-lactide-co-glycolide)-β-ethylene glycol-β-(-lactide-co-glycolide)] has been suggested for use in a controlled release formulation of GLP-1. However like other polymeric systems, the manufacture of triblock copolymer involves complex protocols and inconsistent particulate formation.

Similarly, biodegradable polymers, e.g., poly(lactic-co-glycolic acid) (PLGA), have also been suggested for use in sustained delivery formulations of peptides. However the use of such biodegradable polymers has been disfavored in the art since these polymers generally have poor solubility in water and require water-immiscible organic solvents, e.g., methylene chloride, and/or harsh preparation conditions during manufacture. Such organic solvents and/or harsh preparation conditions are considered to increase the risk of inducing conformational change of the peptide or protein of interest, resulting in decreased structural integrity and compromised biological activity (Choi et al., Pharm. Research, Vol. 21, No. 5, (2004).) Poloxamers have been likewise faulted. (Id.)

The GLP-1 compositions described in the foregoing references are less than ideal for preparing pharmaceutical formulations of GLP's since they tend to trap impurities and/or are otherwise difficult to reproducibly manufacture and administer. Also, GLP analogs are known to induce nausea at elevated concentrations, thus there is a need to provide a sustained drug effect with reduced initial plasma concentrations (Ritzel et al., Diabetologia, 38: 720-725 (1995); Gutniak et al., Diabetes Care, 17(9): 1039-1044 (1994); Deacon et al., Diabetes, 44: 1126-1131 (1995).) Hence, there is a need for GLP-1 formulations which are more easily and reliably manufactured, that are more easily and reproducibly administered to a patient, and that provide for reduced initial plasma concentrations in order to reduce or eliminate unwanted side-effects.

## Summary of the Invention

The invention may be summarized in the following paragraphs as well as the claims.

In one aspect, the invention provides a sustained release liquid pharmaceutical composition comprising:

- a liquid;
- a peptide analogue according to formula (I):

$$[\text{Aib}^{8,35}]\text{hGLP-1(7-36)NH}_2;$$

- a divalent metal and/or divalent metal salt; and

- an acetate salt and/or acetic acid.

In another aspect the invention provides a container comprising the pharmaceutical composition according to any one of the preceding claims.

In a further aspect, the invention provides a method for preparing the pharmaceutical composition, comprising the steps of:

A. combining the liquid, acetate salt and/or acetic acid and the peptide analogue; and
B. adding and dissolving the divalent metal and/or divalent metal salt.

In yet another aspect, the invention provides for the use of the pharmaceutical composition for the treatment of Type II diabetes.

Brief Description of the Drawings

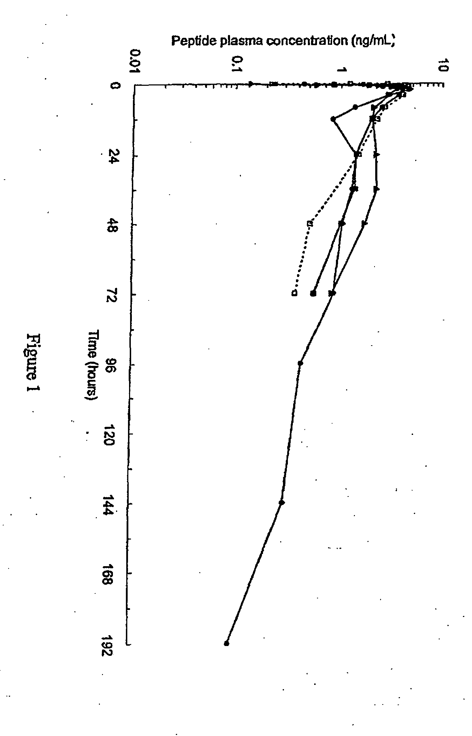

Figure 1 depicts the plasma profiles (median values) obtained after a single subcutaneous (s.c.) administration to dogs of approximately 1mg of [Aib$^{8,35}$]hGLP-1(7-36)NH$_2$. In each case the peptide was administered as an aqueous zinc composition comprising approximately 1% (wt/vol) peptide and having a peptide:Zn molar ratio of approximately 1.5. Filled squares and open squares represent compositions in which the pH is adjusted with NaOH as described herein; filled triangles represent a composition in which the pH was not adjusted with NaOH; filled circles represent a composition in buffered with AcOH/AcO-.

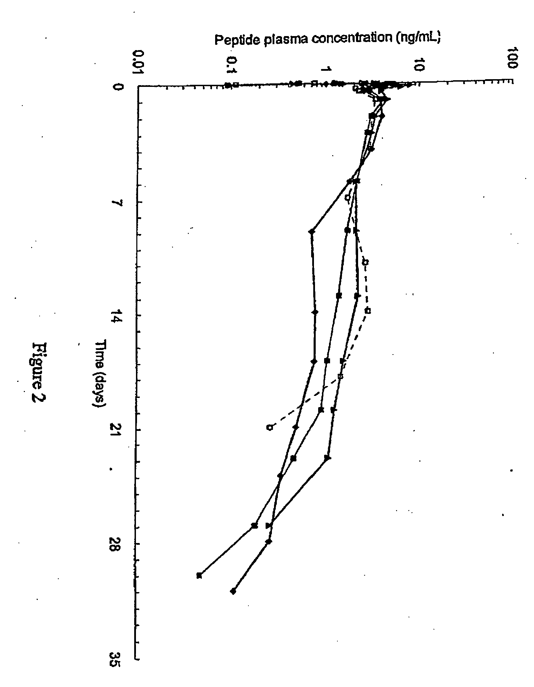

Figure 2 depicts the plasma profiles (median values) obtained after a single subcutaneous (s.c.) administration to dogs of approximately 15 mg of [Aib$^{8,35}$]hGLP-1(7-36)NH$_2$. In each case the peptide was administered as an aqueous zinc composition comprising approximately 10% (wt/vol) peptide and having a peptide:Zn molar ratio of approximately 1.5. Filled squares and open squares represent compositions in which the pH is adjusted with NaOH as described herein; filled triangles represent a composition in which the pH was not adjusted with NaOH; filled circles represent a composition in buffered with AcOH/AcO-.

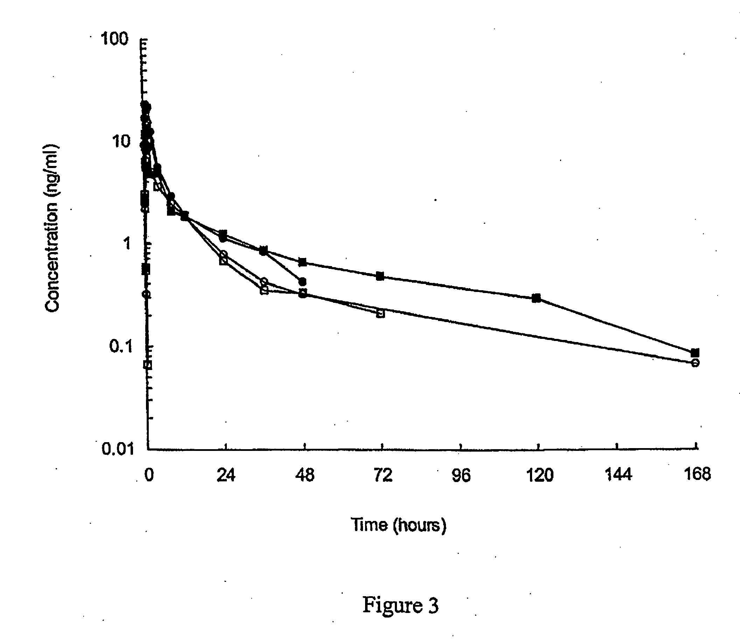

Figure 3 depicts the plasma profiles (median values) obtained after a single subcutaneous (s.c.) administration to dogs of approximately 1 mg of [Aib$^{8,35}$]hGLP-1(7-36)NH$_2$. In each case the peptide was administered as an semisolid aqueous zinc composition as follows: solid circle: about 5% (wt/vol) peptide, peptide:Zn molar ratio about 5.4:1, no pH adjustment; open circle: about 10% (wt/vol) peptide, peptide:Zn molar ratio about 5.4:1, no pH adjustment; open square: about 10% (wt/vol) peptide, peptide:Zn molar ratio about 5.4:1, pH adjusted with NaOH; solid square: about 10% (wt/vol) peptide, peptide:Zn molar ratio about 4:1, pH adjusted with NaOH.

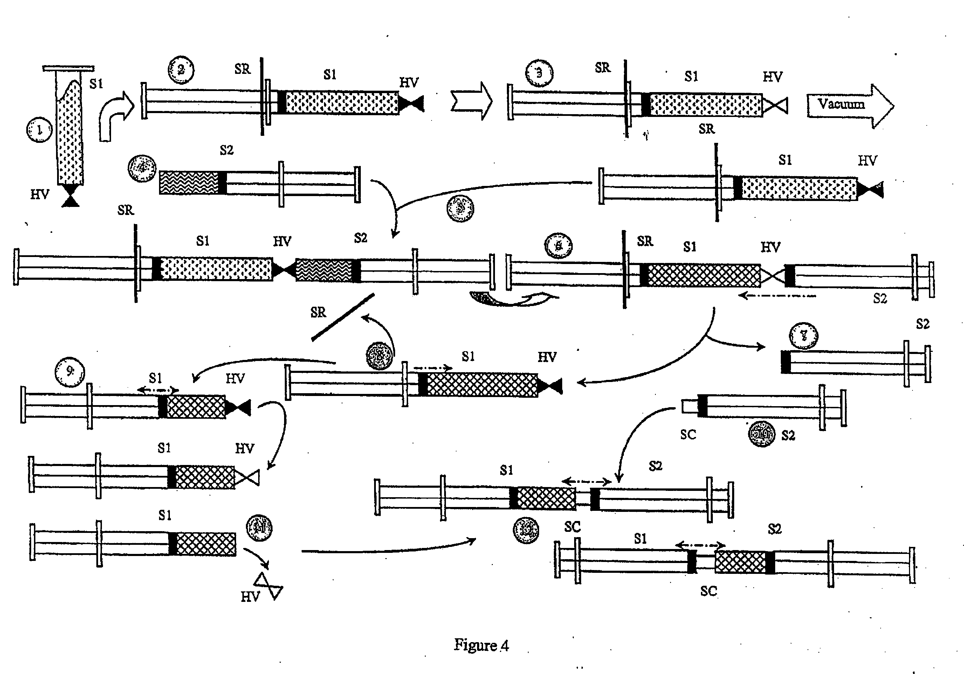

Figure 4 provides a schematic presentation of various devices useful in preparing certain formulations of the present invention.

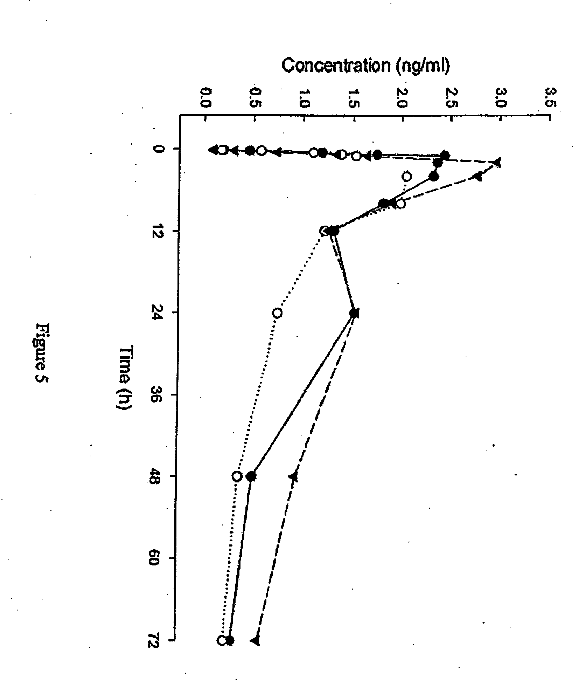

Figure 5 depicts the plasma profiles (median values) obtained after a single subcutaneous (s.c.) administration to dogs of approximately 1 mg of [Aib$^{8,35}$]hGLP-1(7-36)NH$_2$. The peptide was administered as an aqueous zinc composition having a peptide concentration of about 2%, and a peptide:Zn molar ratio of about 1.5:1.

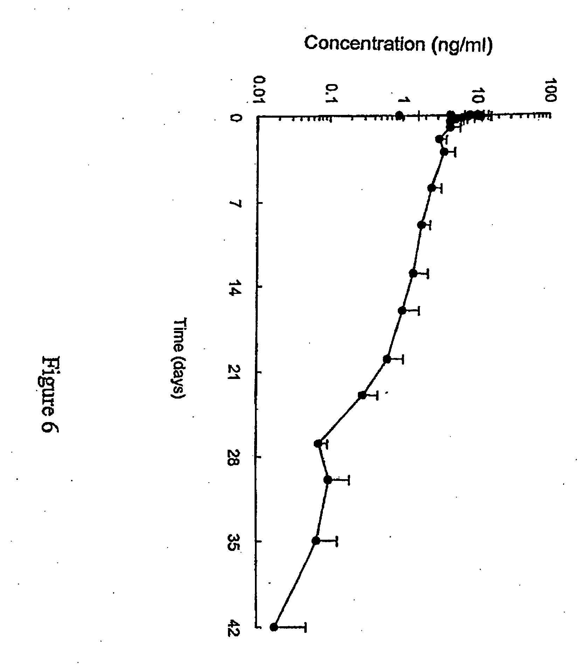

Figure 6 depicts the plasma profiles (median values) obtained after a single subcutaneous (s.c.) administration to dogs of approximately 15 mg of [Aib$^{8,35}$]hGLP-1(7-36)NH$_2$. The peptide was administered as a semisolid zinc composition having a peptide concentration of about 25%, and a peptide:Zn molar ratio of about 4:1.

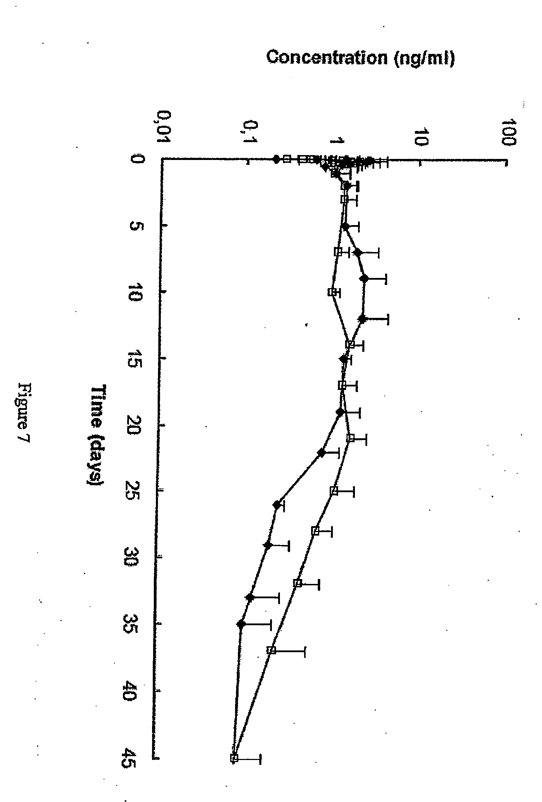

Figure 7 depicts the plasma profiles (median values) obtained after a single subcutaneous (s.c.) administration to dogs of approximately 15 mg of [Aib$^{8,35}$]hGLP-1(7-36)NH$_2$. The peptide was administered as a semisolid zinc composition having a peptide concentration of about 23%, and a peptide:Zn molar ratio of about 1.5:1.

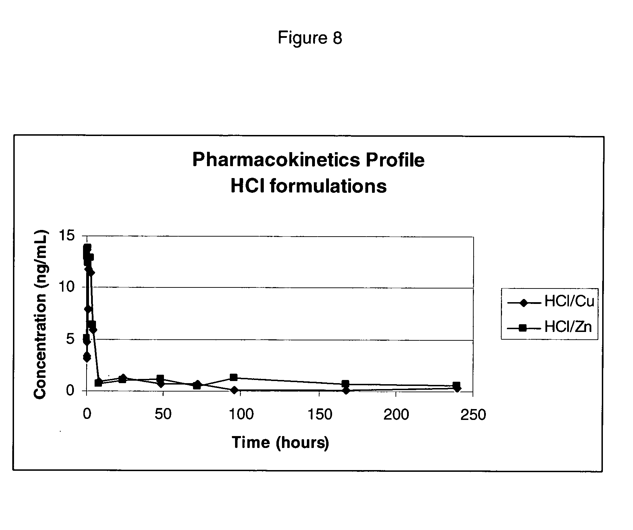

Figure 8 depicts the full time course plasma profiles (median values) obtained after a single subcutaneous (s.c.) administration to rats of 0.3mg of (3 μL of 10% solution) of the GLP-1 analog HCl salt test formulations:

(1) (Aib$^{8,35}$)hGLP-1(7-36)NH$_2$ HCl salt with CuCl$_2$: the molar ratio of (Aib$^{8,35}$)hGLP-1(7-36)NH$_2$/CuCl$_2$ is 1.5:1. The peptide concentration is 10% (30 mM) in water (w/w) with approximately pH5.5.

(2) (Aib$^{8,35}$)hGLP-1(7-36)NH$_2$ HCl salt with ZnCl$_2$: the molar ratio of (Aib$^{8,35}$)hGLP-1(7-36)NH$_2$/ZnCl$_2$ is 1.5:1. The peptide concentration is 10% (30 mM) in water (w/w) with approximately pH5.5.

Figure 9 depicts the full time course plasma profiles (median values) obtained after a single subcutaneous (s.c.) administration to rats of 0.3mg of (3 μL of 10% solution) of the GLP-1 analog acetate salt test formulation:

(Aib$^{8,35}$)hGLP-1(7-36)NH$_2$ acetate salt with ZnCl$_2$: the molar ratio of (Aib$^{8,35}$)hGLP-1(7-36)NH$_2$/ZnCl$_2$ is 1.5:1. The peptide concentration is 10% (30 mM) in water (w/w) with approximately pH5.5.

Figure 10 depicts the early time course plasma profiles (median values) obtained after a single subcutaneous (s.c.) administration to rats of 0.3mg of (3 µL of 10% solution) of the test formulations shown in Figure 8.

Figure 11 depicts the early time course plasma profiles (median values) obtained after a single subcutaneous (s.c.) administration to rats of 0.3mg of (3 µL of 10% solution) of the test formulations shown in Figure 9.

Figure 12 depicts the estimated percentage of (Aib$^{8,35}$)hGLP-1(7-36)NH$_2$ remaining at the injection site of rats after a single subcutaneous (s.c.) administration of 0.3mg of (3 µL of 10% solution) of the three test formulations shown in Figure 8.

## Detailed Description

The invention provides a pharmaceutical composition comprising a GLP-1 analog. Particularly preferred is a GLP-1 analog according to formula (I):

$$(Aib^{8,35})hGLP\text{-}1(7\text{-}36)NH_2$$

$$(I)$$

or a pharmaceutically acceptable salt thereof, wherein the formulation of said composition provides for superior manufacturing, administration, pharmacokinetic and pharmacodynamic properties, as well as attenuated negative side-effects. Preferably the pharmaceutical composition of the invention does not consist of a clear aqueous ZnCl$_2$ solution having pH 4 in which said [Aib$^{8,35}$]hGLP-1(7-36)NH$_2$ is present at a concentration of 4 mg/ml and said ZnCl$_2$ is present at a concentration of 0.5 mg/ml.

One preferred embodiment the invention provides for a pharmaceutical composition having an improved drug release profile, preferably with a reduced initial burst.

The invention further provides a pharmaceutical composition comprising a compound of formula (I) having an extended duration of action.

The invention may be summarized in the following paragraphs as well as the claims.

In one aspect, the invention provides a sustained release liquid pharmaceutical composition comprising:

- a liquid;
- a peptide analogue according to formula (I):

$$[Aib^{8,35}]hGLP\text{-}1(7\text{-}36)NH_2;$$

- a divalent metal and/or divalent metal salt; and
- an acetate salt and/or acetic acid.

Preferably, a portion of the peptide analogue and a portion of the acetate salt are present as a salt form of the peptide analogue.

Preferably, the final pH of the pharmaceutical composition is within the range of 3.5 to 6. More preferably, the final pH of the pharmaceutical composition is within the range of 4 to 5. Even more preferably the final pH of the pharmaceutical composition is 4.5 ± 0.1. The final pH of the composition is the pH of the composition when it is ready to be administered.

Preferably, the molar ratio range of the acetate salt and/or acetic acid to the peptide analogue is approximately 0.5:1 to approximately 10:1. More preferably, the pharmaceutical composition according to claim 5 wherein the molar ratio range of the acetate salt and/or acetic acid to the peptide analogue is approximately 1:1 to approximately 6:1. Even more preferably, the pharmaceutical composition according to claim 6 wherein the molar ratio of the acetate salt and/or acetic acid to the peptide analogue is approximately 3.2:1.

Preferably, the divalent metal and/or divalent metal salt is zinc chloride.

Preferably, the molar ratio range of the peptide analogue to zinc is approximately 6:1 to approximately 1:1. More preferably, the molar ratio of the peptide analogue to zinc is approximately 1.5:1.

Preferably, the concentration of the peptide analogue is about 10 % by weight.

Preferably, the composition is formulated such that the peptide analogue according to formula (I) is released within a subject for at least approximately 1 week.

Preferably, the composition is formulated such that the compound according to formula (I) is released within the subject for at least approximately 1 week, preferably 2 weeks.

Preferably, the composition further comprises a diluent. The diluent is used as solvent or vehicle of suspension.

Preferably, the liquid is selected from sterile water or a sterile water comprising an isotonic agent such as NaCl.

Preferably, the pharmaceutical composition is suitable for parenteral administration. More preferably, the pharmaceutical composition is suitable for administration by injection.

Preferably, the pharmaceutical composition is suitable for storage before use in a state ready to be used and at a temperature of 5 °C for a period of at least one year.

In another aspect the invention provides a container comprising the pharmaceutical composition according to any one of the preceding claims. Preferably, the container is a prefilled syringe.

In another aspect, the invention provides a method for preparing the pharmaceutical composition, comprising the steps of:

> A. combining the liquid, acetate salt and/or acetic acid and the peptide analogue; and
> B. adding and dissolving the divalent metal and/or divalent metal salt.

Preferably, the method further comprises the steps of:

> C. sterile filtrating the composition resulting from Step B; and
> D. filling a container with the composition.

Preferably, before step A, the acetic acid and the sterile water are combined.

More preferably, the method includes a final step of adding further sterile water to the solution.

In another aspect, the invention provides for the use of the pharmaceutical composition for the treatment of Type II diabetes.

In preferred features, the invention also provides for a pharmaceutical composition which precipitates *in vivo* at physiological pH to form an *in situ* deposit for a sustained release drug profile.

A further embodiment of the invention provides for a pharmaceutical composition comprising a compound of formula (I) or a pharmaceutically acceptable salt thereof and a pharmaceutically acceptable carrier or diluent. Preferably said carrier or diluent comprises water.

In preferred features, the invention provides a pharmaceutical composition which comprises a compound or GLP-1 peptide analog prepared with a salt of the peptide or with a mixture of peptide and salt thereof.

Preferably, the salt of the GLP-1 peptide analog in said pharmaceutical composition is selected from the list of pharmaceutically acceptable salts of organic acids, such as those of acetic, lactic, malic, ascorbic, succinic, benzoic, citric, methanesulphonic or toluenesulphonic acids, or pharmaceutically acceptable salts of inorganic acids, such as those of hydrochloric, hydrobromic, hydriodic, sulfuric or phosphoric acids. Pharmaceutically acceptable salts of strong acids, such as hydrochloric acid, are particularly preferred. A strong acid is defined as an acid having a pKA of less than 4.5. Additional preferred peptide salts in said pharmaceutical composition are salts of organic acids such as those of acetic acid or trifluoroacetic acid, lactic, malic, ascorbic, succinic, benzoic, or citric acid.

In one preferred embodiment, the solubility, the pH, and the release profile of the pharmaceutical composition can be modulated by adjusting the molar ratio of GLP-1 analog in salt form to GLP-1 analog not in salt form to extend the release profile and reduce the initial spike in GLP-1 analog concentration.

In a preferred embodiment, the pharmaceutical composition further comprises a divalent metal to lower the water solubility of the composition and thereby extend the release profile while simultaneously reducing the initial burst or spike in plasma concentrations. Preferred divalent metals include zinc and copper. Salt forms of the divalent metals are particularly preferred, including but not limited to chloride and acetate salts of the divalent metals. $CuAc_2$, $CuCl_2$, $ZnAc_2$, and/or $ZnCl_2$ are most preferred. Preferably, the divalent metal and/or divalent metal salts in said pharmaceutical composition is present in a concentration from about 0.0005mg/ml to about 50mg/m. Even more preferably, the divalent metal and/or divalent metal salts in said pharmaceutical composition is present in a concentration from about 0.01 mg/ml to about 0.50 mg/ml. More preferably, said pharmaceutical composition comprises a diluent, wherein said diluent comprises a pharmaceutically acceptable aqueous solution. The diluent may comprise sterile water or a sterile water solution of a salt such as NaCl, as isotonic agent.

The term "isotonic agent" means in this context a salt or any excipient in solution which maintains the same osmotic pressure as blood.

The term "pharmaceutically acceptable" means in this context physiologically well tolerated by a mammal or a human.

In a further embodiment, said pharmaceutical composition further comprises a divalent metal and/or divalent metal salt, wherein the molar ratio of said GLP-1 analog to said divalent metal and/or divalent metal salt in said pharmaceutical composition ranges from approximately 6:1 to approximately 1:1. Preferably, said ratio ranges from approximately 5.5:1 to approximately 1:1. More preferably, said ratio ranges from approximately 5.4:1 to approximately 1.5:1. Even more preferably still, said ratio is approximately 5.4:1, 4.0:1, or 1.5:1. Most preferably, said ratio is approximately 1.5:1. The molar ratio of GLP-1 analogue to divalent metal and/or divalent metal salt means the molar proportion of the peptide analogue in the pharmaceutical composition to the molar proportion of divalent metal and/or divalent metal salt. The molar proportion of the peptide analogue includes any peptide present in the form of a salt of the peptide analogue. What is meant by approximately in this aspect of the invention is a ratio of 1.5:1 $\pm$ 10% each target value, thus expected ratios include ratios encompassing, e.g., 1.35-1.65:0.85-1.15.

Preferably, said pharmaceutical composition comprises an aqueous mixture, suspension or solution, wherein said analog of GLP-1, compound of formula (I), or salt thereof is present at a concentration of approximately 0.5% - 30% (w/w). More preferably the concentration of said GLP-1 analog and/or salt thereof in said aqueous mixture, suspension or solution is approximately 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30% (w/w). More preferably, the concentration of said GLP-1 analog and/or salt thereof in said aqueous solution is approximately 1%, 2%, 3%, 4%, 5%, 6%, 7%, 8%, 9%, 10%, 11%, 14%, 15%, 16%, 19%, 20%, 21%, 22%, 23%, 24%, 25%, 26%, 29%, or 30% (w/w). More preferably still, the concentration of said analog of GLP-1 analog and/or salt thereof in said aqueous solution is approximately 1%, 2%, 3%, 4%, 5%, 6%, 9%, 10%, 11 %, 22%, 23%, 24%, 25%, or 26% (w/w). Even more preferably still, the concentration of said analog of GLP-1 and/or salt thereof in said aqueous solution is approximately 1%, 2%, 3%, 4%, 5%, 6%, 10%, 22%, 23%, 24%, 25%, or 26% (w/w). Still more preferably, the concentration of said analog of GLP-1 and/or salt thereof in said aqueous solution is approximately 1%, 2%, 5%, 10%, 23% or 25% (w/w). By "approximately" is meant the following: for concentrations of about 0.5% to about 4%, $\pm$ 0.5% of the target value is the desired range (for example, 0.5% to 1.5% is approximately 1 %); for target concentrations of about 5% and higher, 20% of the target value is the desired range (for example, 8% to 12% is approximately 10%).

Preferably, the concentration of $[Aib^{8,35}]hGLP-1(7-36)NH_2$, analog of GLP-1, or salt thereof in the pharmaceutical composition is about 1% (weight/volume) and the molar ratio of $[Aib^{8,35}]hGLP-1(7-36)NH_2$ to said divalent metal and/or divalent metal salt is about 1.5:1. More preferably, the concentration of $[Aib^{8,35}]hGLP-1(7-36)NH_2$ or salt thereof in said pharmaceutical composition is about 2% (weight/volume) and the molar ratio of $[Aib^{8,35}]hGLP-1(7-36)NH_2$ or salt thereof to said divalent metal and/or divalent metal salt is about 1.5:1. More preferably still, the concentration of $[Aib^{8,35}]hGLP-1(7-36)NH_2$ or salt thereof in said pharmaceutical composition is about 10% (weight/volume) and the molar ratio of $[Aib^{8,35}]hGLP-1(7-36)NH_2$ or salt thereof to said divalent metal and/or divalent metal salt is about 1.5:1. Most preferably, the concentration of $[Aib^{8,35}]hGLP-1(7-36)NH_2$ or salt thereof in said pharmaceutical composition is about 23% or about 25% (weight/volume) and the molar ratio of $[Aib^{8,35}]hGLP-1(7-36)NH_2$ or salt thereof to said divalent metal and/or divalent metal salt is about 1.5:1.

In a preferred embodiment, the concentration of the analog of GLP-1, $[Aib^{8,35}]hGLP-1(7-36)NH_2$, or salts thereof in the pharmaceutical composition is about 5% (weight/volume) and the molar ratio of the peptide to the divalent metal and/or divalent metal salt is approximately 5.4:1. More preferably, the concentration of $[Aib^{8,35}]hGLP-1(7-36)NH_2$ or salt thereof in said composition is about 5% (weight/volume) and said ratio is approximately 4.0:1. More preferably still, the concentration of $[Aib^{8,35}]hGLP-1(7-36)NH_2$ or salt thereof in said composition is about 10% (weight/volume) and said ratio is approximately 5.4:1. Still further preferably, the concentration of $[Aib^{8,35}]hGLP-1(7-36)NH_2$ or salt thereof in said composition is about 10% (weight/volume) and said ratio is approximately 4.0:1.

Preferably, said divalent metal and/or divalent metal salt is provided as zinc chloride or zinc acetate. More preferably, said zinc acetate is provided as $ZnAC_2.2 H_2O$.

In an alternative embodiment, said divalent metal and/or divalent metal salt is provided as copper chloride or copper acetate.

In one embodiment, the pH of said pharmaceutical composition is adjusted upward using a base. More preferably, said pH adjustment is made using NaOH. More preferably still, the pH of said pharmaceutical composition is adjusted with NaOH such that, when diluted to approximately ½ initial concentration using 0.9% NaCl, a pH value of approximately 5.0 - 5.5 is obtained using direct potentiometric determination.

A preferred embodiment of the invention features a pharmaceutical composition or sustained release formulation, wherein the composition is formulated such that a peptide analog of GLP-1 or salt thereof, e.g., the compound according to formula (I) or salt thereof, is released within a subject in need thereof, e.g., a mammal, preferably a human, for an extended period of time. Preferably said release of said compound extends for at least one hour, more preferably at least 4, 6, 12, or 24 hours. More preferably still, said composition is formulated such that the compound according to formula (I) is released within a subject for at least 36, 48, 60, 72, 84, or 96 hours. More preferably still, said composition is formulated such that the compound according to formula (I) is released within a subject for at least approximately 5, 6, 7, 8, 9, 10, 11, 12, 13, or 14 days. More preferably still, said composition is formulated such that the compound according to formula (I) is released within a subject for at least approximately 2, 3 or 4 weeks.

The term "sustained release" as used herein means a release which results in a measurable serum level of biologically active GLP-1 analog, for a period of at least one week and more preferably for a period of at least two weeks.

In one aspect of the invention, the modulation of the salt content of the GLP-1 peptide analog in said pharmaceutical composition improves the solubility and the stability of the GLP-1 peptide analog in the pharmaceutical composition and furthermore provides an improvement on the *in vivo* release profile by decreasing the initial burst.

The wording "modulation" means in this aspect of the invention adjustment of salt content by adjusting the molar ratio of the GLP-1 analog in salt form to GLP-1 analog not in salt form.

Even more preferably, the peptide salt in said pharmaceutical composition is a salt of hydrochloric or acetic acid, or chlorides or acetates of said peptide of formula (1). In said pharmaceutical composition the acetate or chlorides is present as final molar ratio of acetate or chloride to said compound of formula (1) in ranges from approximately of 0.5:1 to approximately 10:1. More preferably said ratio ranges from approximately 0.8:1 to approximately 9:1. Even more preferably said ratio is approximately 1:1 to approximately 6:1. Most preferably said ratio is approximately 3.0:1 in particular 3.2:1.

In this aspect of the invention, the molar ratio of acetate or chloride to peptide means the molar proportion of acetate ($CH_3COO^-$) or chloride ($Cl^-$) in the pharmaceutical composition to the molar proportion of the peptide in the pharmaceutical composition. In example for a molar ratio of 3:1 in the pharmaceutical composition, acetate is three times the molar content of the peptide in proportion. This is a stoichiometric ratio of a compound compared to the other.

The molar proportion of acetate and the molar proportion of the peptide include any acetate and peptide present in the form of a salt of the peptide analogue. The concentration of acetate in the peptide as initially prepared may vary, depending on the method used to prepare the peptide. The amount of acetate added to the formulation must therefore be adapted such that the appropriate final ratio of peptide to acetate is obtained.

In another aspect, the invention relates to methods of using such compositions to treat mammals.

The wording "approximately" means in this preferred embodiment a ratio of 1.5:1 $\pm$ 10% each target value, thus expected ratios include ratios encompassing, e.g., 1.35-1.65:0.85-1.15.

In additional preferred aspects of the invention, the pharmaceutical composition pH is adjusted by modulation of the acetate content of the composition. Preferably, the pH ranges of said pharmaceutical composition is from pH 3 to pH 6. More preferably said pH ranges of said pharmaceutical composition is from pH 3.5 to 5.5. Most preferably said pH ranges of said pharmaceutical composition is from pH 4.2 to pH 4.6.

Preferably, to acidify the pharmaceutical composition the acetate content may be increased by adding acetic acid.

In one embodiment, the pH of the said pharmaceutical composition may be increased starting from a peptide salt of an analog of GLP-1 having a low acetate or no acetate content by modulation of acetate content.

In preferred embodiments, adjustment of the pH in the final pharmaceutical composition by modulation of acetate or chloride content allows modulation of parameters such as, the peptide concentration, the zinc concentration, the chemical stability, the physical stability and *in vivo* release profile by decreasing the initial burst.

In one aspect of the invention, Zn or Cu content is fixed, pH is controlled by modulating the acetate content. Increased content of acetate shows an improvement on the solubility and the physical stability and decreased content of acetate shows an increasing effect on the pH and decreasing effect on the $C_{max}$.

In preferred embodiments, said pharmaceutical composition comprises an aqueous mixture, suspension or solution.

The present invention also provides for a method of eliciting a GLP-1 agonist effect, said method comprising contacting a receptor of the GLP-1 (7-36)$NH_2$ ligand with a GLP-1 analog or salt thereof, directly or indirectly.

In the foregoing method, said receptor of the GLP-1(7-36)$NH_2$ ligand is present in an animal subject, preferably a primate, more preferably a human being. Thus, in this embodiment the present invention provides a method of eliciting an agonist effect from a GLP-1 receptor in a subject in need thereof which comprises administering to said subject a composition of the instant invention, wherein said composition comprises an effective amount of a GLP-1 analog or a pharmaceutically acceptable salt thereof.

In a preferred aspect of the foregoing method, said subject is a human afflicted with, or at risk of developing, a disease or condition selected from the group consisting of Type I diabetes, Type II diabetes, gestational diabetes, obesity, excessive appetite, insufficient satiety, and metabolic disorder. Preferably said disease is Type I diabetes or Type II diabetes.

In another more preferred aspect of the foregoing method, said subject is a human afflicted with, or at risk of developing, a disease selected from the group consisting of Type I diabetes, Type II diabetes, obesity, glucagonomas, secretory disorders of the airway, arthritis, osteoporosis, central nervous system disease, restenosis, neurodegenerative disease, renal failure, congestive heart failure, nephrotic syndrome, cirrhosis, pulmonary edema, hypertension, and disorders

wherein the reduction of food intake is desired, a disease or disorder of the central nervous system, (e.g., through modulation of neurogenesis, and e.g., Parkinson's Disease, Alzheimer's Disease, Huntington's Disease, ALS, stroke, ADD, and neuropsychiatric syndromes), irritable bowel syndrome, myocardial infarction (e.g., reducing the morbidity and/or mortality associated therewith), stroke, acute coronary syndrome (e.g., characterized by an absence of Q-wave) myocardial infarction, post-surgical catabolic changes, hibernating myocardium or diabetic cardiomyopathy, insufficient urinary sodium excretion, excessive urinary potassium concentration, conditions or disorders associated with toxic hypervolemia, (e.g., renal failure, congestive heart failure, nephrotic syndrome, cirrhosis, pulmonary edema, and hypertension), polycystic ovary syndrome, respiratory distress, nephropathy, left ventricular systolic dysfunction, (e.g., with abnormal left ventricular ejection fraction), gastrointestinal disorders such as diarrhea, postoperative dumping syndrome and irritable bowel syndrome, (i.e., via inhibition of antro-duodenal motility), critical illness polyneuropathy (CIPN), systemic inflammatory response syndrome (SIRS), dyslipidemia, organ tissue injury caused by reperfusion of blood flow following ischemia, and coronary heart disease risk factor (CHDRF) syndrome.

In an additional aspect of the invention, the invention features a method of converting liver stem/progenitor cells into functional pancreatic cells, of preventing beta-cell deterioration and of stimulating beta-cell proliferation, of suppressing plasma blood levels of norepinepherine, of inducing an inotropic response and of increasing cardiac contractility, of improving nutrition via a non-alimentary route, (e.g., via intravenous, subcutaneous, intramuscular, peritoneal, or other injection or infusion rout), of pre-treating a subject to undergo an endoscopic procedures, and of modulating triglyceride levels, in a subject in need thereof, said method comprising administering to said subject a formulation of the present invention comprising an effective amount of a compound of formula (I) or a pharmaceutically acceptable salt thereof. Preferably said subject is a mammalian animal, more preferably a primate, more preferably still a human being.

A preferred GLP-1 peptide, to be utilized as a peptide salt of the invention, is denoted herein by the following format, e.g., $(Aib^{8,35})hGLP-1(7-36)NH_2$, with the substituted amino acids from the natural sequence placed between the first set of parentheses (e.g., $Aib^{8,35}$ denotes that Aib is substituted for $Ala^8$ and $Gly^{35}$ in hGLP-1). Aib is the abbreviation for $\alpha$-aminoisobutyric acid. The abbreviation GLP-1 means glucagon-like peptide-1; hGLP-1 means human glucagon-like peptide-1. The numbers between the second set of parentheses refer to the number of amino acids present in the peptide (e.g., hGLP-1 (7-36) refers to amino acids 7 through 36 of the peptide sequence for human GLP-1). The sequence for hGLP-1 (7-37) is listed in Mojsov, S., Int. J. Peptide Protein Res,. 40, 1992, pp. 333-342. The designation "$NH_2$" in hGLP-1 (7-36)$NH_2$ indicates that the C-terminus of the peptide is amidated. hGLP-1(7-36) means that the C-terminus is the free acid. In hGLP-1 (7-38), residues in positions 37 and 38 are Gly and Arg, respectively, unless otherwise indicated.

Particularly preferred GLP-1 peptide analogs used in this invention are in the form of pharmaceutically acceptable salts. Examples of such salts include, but are not limited to, those formed with organic acids (e.g., acetic, lactic, maleic, citric, malic, ascorbic, succinic, benzoic, methanesulfonic, toluenesulfonic, or pamoic acid), inorganic acids (e.g., hydrochloric acid, sulfuric acid, or phosphoric acid), and polymeric acids (e.g., tannic acid, carboxymethyl cellulose, polylactic, polyglycolic, or copolymers of polylactic-glycolic acids). A typical method of making a salt of a peptide of the present invention is well known in the art and can be accomplished by standard methods of salt exchange. Accordingly, the TFA salt of a peptide of the present invention (the TFA salt results from the purification of the peptide by using preparative HPLC, eluting with TFA containing buffer solutions) can be converted into another salt, such as an acetate salt by dissolving the peptide in a small amount of 0.25 N acetic acid aqueous solution. The resulting solution is applied to a semi-prep HPLC column (Zorbax, 300 SB, C-8). The column is eluted with (1) 0.1 N ammonium acetate aqueous solution for 0.5 hrs., (2) 0.25N acetic acid aqueous solution for 0.5 hrs. and (3) a linear gradient (20% to 100% of solution B over 30 min.) at a flow rate of 4 ml/min (solution A is 0.25N acetic acid aqueous solution; solution B is 0.25N acetic acid in acetonitrile/water, 80:20). The fractions containing the peptide are collected and lyophilized to dryness.

As is well known to those skilled in the art, the known and potential uses of GLP-1 are varied and multitudinous (See, Todd, J.F., et al., Clinical Science, 1998, 95, pp. 325-329; and Todd, J.F. et al., European Journal of Clinical Investigation, 1997, 27, pp.533-536). Thus, the administration of the compounds of this invention for purposes of eliciting an agonist effect can have the same effects and uses as GLP-1 itself. These varied uses of GLP-1 may be summarized as follows, treatment of: Type I diabetes, Type II diabetes, obesity, glucagonomas, secretory disorders of the airway, metabolic disorder, arthritis, osteoporosis, central nervous system diseases, restenosis, neurodegenerative diseases, renal failure, congestive heart failure, nephrotic syndrome, cirrhosis, pulmonary edema, hypertension, disorders wherein the reduction of food intake is desired, as well as the various other conditions and disorders discussed herein. Accordingly, the present invention includes within its scope pharmaceutical compositions as defined herein comprising, as an active ingredient, a compound of formula (I).

The dosage of active ingredient in the formulations of this invention may be varied; however, it is necessary that the amount of the active ingredient be such that a suitable dosage is obtained. The selected dosage depends upon the desired therapeutic effect, on the route of administration, and on the duration of the treatment, and normally will be determined by the attending physician. In general, an effective dosage for the activities of this invention is in the range of $1 \times 10^{-7}$ to 200 mg/kg/day, preferably $1 \times 10^{-4}$ to 100 mg/kg/day, which can be administered as a single dose or divided into multiple doses.

The formulations of this invention are preferably administered parenterally, e.g., intramuscularly, intraperitoneally, intravenously, subcutaneously, and the like.

Preparations according to this invention for parenteral administration include sterile aqueous or non-aqueous solutions, suspensions, gels, or emulsions, provided that the desired *in vivo* release profile is achieved. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Synthesis of Peptides

Peptides useful for practicing the present invention can be and were prepared by standard solid phase peptide synthesis. See, e.g., Stewart, J.M., et al., Solid Phase Synthesis (Pierce Chemical Co., 2d ed. 1984).

The following examples describe synthetic methods that can be and were used for making peptides with which the instant invention may advantageously be practiced, which synthetic methods are well-known to those skilled in the art. Other methods are also known to those skilled in the art. The examples are provided for the purpose of illustration and are not meant to limit the scope of the present invention in any manner.

Said peptides such as GLP-1 analog can be obtained with different synthesis known to those skilled in the art which may comprise final precipitation of the peptide, freeze-drying process, vacuum drying or other drying processes known in the art. Ion exchange chromatography, osmotic exchange of buffer and difiltration could be suitable methods in this invention to purify or select the peptide in different salt form.

Boc-βAla-OH, Boc-D-Arg(Tos)-OH and Boc-D-Asp(OcHex) were purchased from Nova Biochem, San Diego, California. Boc-Aun-OH was purchased from Bachem, King of Prussia, PA. Boc-Ava-OH and Boc-Ado-OH were purchased from Chem-Impex International, Wood Dale, IL. Boc-2Nal-OH was purchased from Synthetech, Inc. Albany, OR.

The full names for other abbreviations used herein are as follows: Boc for t-butyloxycarbonyl, HF for hydrogen fluoride, Fm for formyl, Xan for xanthyl, Bzl for benzyl, Tos for tosyl, DNP for 2,4-dinitrophenyl, DMF for dimethylformamide, DCM for dichloromethane, HBTU for 2-(1H-Benzotriazol-1-yl)-1,1,3,3-tetramethyl uronium hexafluorophosphate, DIEA for diisopropylethylamine, HOAc for acetic acid, TFA for trifluoroacetic acid, 2ClZ for 2-chlorobenzyloxycarbonyl, 2BrZ for 2-bromobenzyloxycarbonyl, OcHex for O-cyclohexyl, Fmoc for 9-fluorenylmethoxycarbonyl, HOBt for N-hydroxybenzotriazole; PAM resin for 4-hydroxymethylphenylacetamidomethyl resin; Tris for Tris(hydroxymethyl)aminomethane; and Bis-Tris for Bis(2-hydroxyethyl)amino-tris(hydroxymethyl)methane (i.e., 2-Bis(2-hydroxyethyl)amino-2-(hydroxymethyl)-1,3-propanediol). The term "halo" or "halogen" encompasses fluoro, chloro, bromo and iodo.

Unless defined otherwise, all technical and scientific terms used herein have the same meaning as commonly understood by one of ordinary skill in the art to which this invention belongs. Also, all publications, patent applications, patents and other references mentioned herein are incorporated by reference.

Examples 1

$$(Aib^{8,35})hGLP\text{-}1(7\text{-}36)NH_2$$

A detailed synthesis procedure for $(Aib^{8,35})hGLP\text{-}1(7\text{-}36)NH_2$ has been provided in International Patent Publication No. WO 00/34331 (PCT/EP99/09660), the contents of which are incorporated herein in their entirety. Briefly, the compound was synthesized on an Applied Biosystems (Foster City, CA) model 430A peptide synthesizer which was modified to do accelerated Boc-chemistry solid phase peptide synthesis. See Schnolzer, et al., Int. J. Peptide Protein Res., 40:180 (1992). 4-methylbenzhydrylamine (MBHA) resin (Peninsula, Belmont, CA) with the substitution of 0.91 mmol/g was used. The Boc amino acids (Bachem, CA, Torrance, CA; Nova Biochem., LaJolla, CA) were used with the following side chain protection: Boc-Ala-OH, Boc-Arg(Tos)-OH, Boc-Asp(OcHex)-OH, Boc-Tyr(2BrZ)-OH, Boc-His (DNP)-OH, Boc-Val-OH, Boc-Leu-OH, Boc-Gly-OH, Boc-Gln-OH, Boc-Ile-OH, Boc-Lys(2ClZ)-OH, Boc-Thr(Bzl)-OH, Boc-Ser(Bzl)-OH, Boc-Phe-OH, Boc-Aib-OH, Boc-Glu(OcHex)-OH and Boc-Trp(Fm)-OH. The Boc groups were removed by treatment with 100% TFA for 2 x 1 min. Boc amino acids (2.5 mmol) were pre-activated with HBTU (2.0 mmol) and DIEA (1.0 ml) in 4 ml of DMF and were coupled without prior neutralization of the peptide-resin TFA salt. Coupling times were 5 min. except for the Boc-Aib-OH residues and the following residues, Boc-Lys(2ClZ)-OH and Boc-His(DNP)-OH wherein the coupling times were 2 hours.

At the end of the assembly of the peptide chain, the resin was treated with a solution of 20% mercaptoethanol/ 10% DIEA in DMF for 2 x 30 min. to remove the DNP group on the His side chain. The N-terminal Boc group was then removed by treatment with 100% TFA for 2 x 2 min. After neutralization of the peptide-resin with 10% DIEA in DMF (1 x 1 min), the formyl group on the side chain of Trp was removed by treatment with a solution of 15% ethanolamine/ 15% water/ 70% DMF for 2 x 30 min. The peptide-resin was washed with DMF and DCM and dried under reduced pressure. The final cleavage was done by stirring the peptide-resin in 10 ml of HF containing 1 ml of anisole and dithiothreitol (24 mg) at 0°C for 75 min. HF was removed by a flow of nitrogen. The residue was washed with ether (6 x 10 ml) and extracted with 4N HOAc (6 x 10 ml).

The peptide mixture in the aqueous extract was purified on reverse-phase preparative high pressure liquid chromatography (HPLC) using a reverse phase VYDAC® $C_{18}$ column (Nest Group, Southborough, MA). The column was eluted with a linear gradient (20% to 50% of solution B over 105 min.) at a flow rate of 10 ml/min (Solution A = water containing 0.1% TFA; Solution B = acetonitrile containing 0.1% of TFA). Fractions were collected and checked on analytical HPLC. Those containing pure product were combined and lyophilized to dryness. In one example of synthesis of this compound, 135 mg of a white solid was obtained. Purity was 98.6% based on analytical HPLC analysis. Electrospray mass spectrometer (MS(ES))S analysis gave the molecular weight at 3339.7 (in agreement with the calculated molecular weight of 3339.7).

Example 2

Formulation Procedures I

2.1 Materials, Stock Solutions, Calculations

A) Materials: $ZnCl_2$, NaOH pellets, and hydrochloric acid, 35%, were obtained from Panreac Quimica, Barcelona, Spain. WFI (sterile water for injection/irrigation) was obtained from B. Braun Medical, Barcelona, Spain.

B) Stock solutions

(i) $ZnCl_2$, pH=3:

1. With stirring, add 35%HCl to WFI to achieve pH=3.
2. In a volumetric flask, transfer a weighed amount of $ZnCl_2$. With stirring, add pH=3 HCl to achieve a final concentration of approximately 1-4 mg $ZnCl_2$/ml.

(ii) $ZnCl_2$, pH=2:

1. With stirring, add 35%HCl to WFI to achieve pH=2.
2. In a volumetric flask, transfer a weighed amount of $ZnCl_2$. With stirring, add pH=2 HCl to achieve a final concentration of approximately 4-12 mg $ZnCl_2$/ml.

(iii) NaOH, 0.1 to 10 mg/ml:

1. In a volumetric flask, transfer a weighed amount of NaOH. With stirring, add WFII to achieve a final concentration of approximately 0.1-10 mg NaOH/ml.

(iv) Freeze-dried 20 mg aliquot (Aib[8,35])HGLP-1(7-36)NH_2/vial:

1. Prepare a 0.04% (v/v) dilution of acetic acid and WFI.
2. In a volumetric flask, transfer a weighed amount of (Aib[8,35])HGLP-1(7-36)NH_2 (acetate salt). With stirring, add sufficient 0.04% acetic acid to bring the final concentration to 20 mg (Aib[8,35])HGLP-1(7-36)NH_2/ml. Following filter sterilization using 0.45 micron filters, 1 ml aliquots of the solution were transferred to lyophilization vials, freeze dried and the dried product stored at -22°C.

(v) Freeze-dried 50 mg aliquot (Aib[8,35])HGLP-1(7-36)NH_2/vial:

1. Prepare a 0.1 % (v/v) dilution of acetic acid and WFI.
2. In a volumetric flask, transfer a weighed amount of $(Aib^{8,35})HGLP-1(7-36)NH_2$ (acetate salt). With stirring, add sufficient 0.1 % acetic acid to bring the final concentration to 50 mg $(Aib^{8,35})HGLP-1(7-36)NH_2$/ml. Following filter sterilization, 1 ml aliquots of the solution are transferred to lyophilization vials and freeze dried.

C) Calculations

(i) To determine the total weight / volume of excipient (E) for a composition:

$$E = (A \times 100/T) - (A/P)$$

wherein:

E = excipient in mg
A = content of pure peptide (mg);
T = target concentration of the composition; e.g., 2 if target is 2%; and
P = concentration of pure peptide (mg peptide/100 mg formulation)

With respect to the total volume of excipient, the assumption that 1 ml = 1 g is applied.
(ii) To determine the volume/weight (W) of $ZnCl_2$ to add to each ml or g of composition solution:

a) W=100% E for compositions in which no pH adjustment is made;
b) W=80% E for liquid formulations in which the peptide is about 1%, or about 2% or up to about 10% and the pH is adjusted using a base;
c) W=50% E for semi-solid or gel formulations in which the peptide is about 1%, or about 2% or up to about 10% and the pH is adjusted using a base;
d) W=66.66% E for semi-solid or gel formulations in which the peptide is about 25% and the pH is adjusted using a base;
e) W=90% E for formulations in which the peptide is reconstituted from a freeze-dried preparation and the pH is adjusted using a base.

(iii) To determine the volume/weight (W) of NaOH to add to each ml or g of composition solution:

a) W=20% E for formulations in which the peptide is about 1%, or about 2% or up to about 10% and the pH is adjusted using a base;
b) W=50% E for semi-solid or gel formulations in which the peptide is about 1%, or about 2% or up to about 10% and the pH is adjusted using a base;
c) W=33.33% E for semi-solid or gel formulations in which the peptide is about 25% and the pH is adjusted using a base;
d) W=10% E for formulations in which the peptide is reconstituted from a freeze-dried preparation and the pH is adjusted using a base.

(iv). To determine the concentration of $ZnCl_2$ (mg/ml or mg/g) to be used in each composition:

$$[ZnCl_2] = (136.29 \times A)/(W \times 3339.76 \times R)$$

wherein:

A = content of pure peptide (mg).
R = molar ratio of peptide/Zn
R=1.5 for formulations in which the peptide is about 1%, or about 2% or about 10% or up to about 23%;
R=4.0 formulations in which the peptide is about 25%; and
W = weight (g) or volume (ml) of $ZnCl_2$ solution to add to each g or ml of composition solution.

2.2 Preparation of compositions with 1-10% freeze-dried peptide and $ZnCl_2$, no pH adjustment

As used herein, a formulation comprising a percentage of peptide describes a formulation comprising a weight of peptide per total weight of the composition, e.g., 1% peptide, describes a formulation comprising 1 g of peptide per 100g of total composition. Formulations comprising about 1%, or about 2% up to about 10% peptide were prepared as follows. Freeze-dried samples of $(Aib^{8,35})HGLP-1(7-36)NH_2$ prepared as described were thoroughly mixed with a $ZnCl_2$ stock solution pH 3 at 100% of the total excipient volume and [peptide:Zn] = 1.5:1.

A) 1% compositions are prepared by mixing 20 mg freeze-dried $(Aib^{8,35})HGLP-1(7-36)NH_2$ (see 2.1 B (iv) above) with 2 ml of $ZnCl_2$ solution (0.272 mg/ml; see 2.1 B (i) above)
B) 2% compositions are prepared by mixing 20 mg freeze-dried $(Aib^{8,35})HGLP-1(7-36)NH_2$ (see 2.1 B (iv) above) with 1 ml of $ZnCl_2$ solution (0.544 mg/ml; see 2.1 B (i) above)
C) 10% compositions are prepared by mixing 50 mg freeze-dried $(Aib^{8,35})HGLP-1(7-36)NH_2$ (see 2.1 B (v) above) with 0.45 ml of $ZnCl_2$ solution (3.023 mg/ml; see 2.1 B (i) above)

Freeze-dried peptides and solutions were allowed to equilibrate to room temperature. The designated volume of $ZnCl_2$ solution was injected into the vial containing the freeze-dried peptide and hydration allowed to proceed for about 2 minutes for 1% or 2% peptide compositions to about 60 minutes for 10% peptide composition, or until all freeze-dried peptide is completely hydrated and the solution is free of clumps of peptide. Following hydration, the reconstituted peptide is shaken for approximately 1 minute.
The appropriate amount of dissolved peptide may be removed for dosing, e.g., 100 ul of a 1% peptide solution prepared as per A above equates to a 1 mg dose, 50 ul of a 2% peptide solution prepared as per B above equates to a 1 mg dose, 150 ul of a 10% peptide solution prepared as per C above equates to a 15 mg dose, etc.
Using the teachings of the instant application, one skilled in the art could vary the amounts of peptide and $ZnCl_2$ to achieve compositions other than the 1 %, 2% and 10% compositions detailed below as well as desired dosages.

2.3 Preparation of compositions with 1-10% freeze-dried peptide and $ZnCl_2$, with a pH adjustment

Formulations comprising about 1%, or about 2% up to about 10% peptide were prepared as follows. Freeze-dried samples of $(Aib^{8,35})HGLP-1(7-36)NH_2$ prepared as described were thoroughly mixed with a $ZnCl_2$ stock solution pH 3 at 90% of the total excipient volume. The desired pH of the solution is reached by the addition of diluted NaOH solution.

A) 1% compositions are prepared by mixing 20 mg freeze-dried $(Aib^{8,35})HGLP-1(7-36)NH_2$ (see 2.1 B (iv) above) with 1.8 ml of $ZnCl_2$ solution (see 2.1 B (i) above)
B) 2% compositions are prepared by mixing 20 mg freeze-dried $(Aib^{8,35})HGLP-1(7-36)NH_2$ (see 2.1 B (iv) above) with 0.9 ml of $ZnCl_2$ solution (see 2.1 B (i) above)
C) 10% compositions are prepared by mixing 50 mg freeze-dried $(Aib^{8,35})HGLP-1(7-36)NH_2$ (see 2.1 B (v) above) with 0.40 ml of $ZnCl_2$ solution (see 2.1 B (i) above)

To the above solutions, add the necessary volume (10% of total volume of excipient) of diluted NaOH solution to achieve the target concentration and pH. For example, to each:

1% composition: Add 0.2 ml of NaOH solution of proper concentration
2% composition: Add 0.1 ml of NaOH solution of proper concentration
10% composition: Add 0.05 ml of NaOH solution of proper concentration

Using the teachings of the instant application, one skilled in the art could vary the amounts of peptide and $ZnCl_2$ to achieve compositions other than the 1 %, 2% and 10% compositions detailed below.

2.4 Preparation of liquid compositions with 1-10% peptide and $ZnCl_2$, no pH adjustment

Liquid formulations comprising about 1%, or about 2% up to about 10% peptide were prepared as follows. Samples of $(Aib^{8,35})HGLP-1(7-36)NH_2$ were weighed and mixed with a $ZnCl_2$ stock solution pH 3 to achieve the target concentration of 1%, 2%, up to 10% peptide. Following mixing, the composition is filter sterilized and stored until use.

2.5 Preparation of liquid compositions with 1-10% peptide and $ZnCl_2$, pH adjustment

Liquid formulations comprising about 1%, or about 2% up to about 10% peptide were prepared as follows.

Samples of (Aib$^{8,35}$)HGLP-1(7-36)NH$_2$ were weighed and thoroughly mixed with a ZnCl$_2$ stock solution pH 3 at 80% of the total excipient volume. The zinc solution may be either ZnCl$_2$ or ZnAc$_2$•2H20. The desired pH of the solution is reached by the addition of diluted NaOH solution. Preparations C5 to C13 were prepared using this method.

Using the teachings of the instant application, one skilled in the art could vary the amounts of peptide and ZnCl$_2$ to achieve compositions other than the 1 %, 2% and 10% described herein.

2.6 Preparation of semi-solid/gel compositions with 25% peptide and ZnCl$_2$, no

pH adjustment

Semi-solid or gel formulations comprising about 25% peptide were prepared as follows. Samples of (Aib$^{8,35}$) HGLP-1(7-36)NH$_2$ were weighed and thoroughly mixed with a ZnCl$_2$ stock solution pH 2 at 66.66% of the total excipient volume.The zinc solution may be either ZnCl$_2$ or ZnAc$_2$·2H$_2$0. Preparations C1 and C 2 were prepared using this method.

More specifically, the semi-solid or gel compositions were prepared using a "push-pull" mixing method:

a) The desired amount of peptide was weighed into the barrel of a disposable syringe **S1** previously fitted with a special two-way hand valve **HV** (I.D.=0.5 mm) and tubing was placed inside the syringe Luer hole;
b) The syringe plunger was secured with a stainless steel rod **SR;**
c) **HV** in **S1** was connected to a vacuum source and **HV** was opened. After 10 min **HV** was closed;
d) The Zinc solution was accurately weighed into the barrel of a second disposable syringe **S2;**
e) **S2** was then connected to the free part of **HV;**
f) **HV** was opened and the solvent was pulled by the vacuum into the barrel containing the peptide powder **S1**;
g) **HV** was closed and the solvent syringe **S2** was removed, thus hydrating the peptide powder in **S1**;
h) **SR** was removed and the syringe plunger was slowly released;
i) The syringe plunger is moved (push and pull), without opening **HV,** so that the powder mass is fully soaked by solvent;
j) A two-way stainless connector **SC** (I.D.=1.0 mm) was placed in syringe **S2** with the tubing placed inside the syringe Luer hole, and its plunger was pushed to the end;
k) **HV** in **S1** was opened to vent the vacuum and then **HV** was removed. The syringe plunger was moved so that air in the syringe barrel was minimized; and
l) **S1** and **S2** were connected by **SC** and the composition was kneaded from **S1** to **S2** through **SC.**

Using the teachings of the instant application, one skilled in the art could vary the amounts of peptide and ZnCl$_2$ to achieve compositions other than the 25% described herein.

2.7 Preparation of semi-solid/gel compositions with 25% peptide and ZnCl$_2$,

pH adjustment

Semi-solid or gel formulations comprising about 25% peptide were prepared as follows. Samples of (Aib$^{8,35}$) HGLP-1(7-36)NH$_2$ were weighed and thoroughly mixed with a ZnCl$_2$ stock solution pH 2 at 66.66% of the total excipient volume. The zinc solution may be either ZnCl$_2$ or ZnAc$_2$•2H$_2$0. The desired pH of the solution is reached by the addition of diluted NaOH solution. In this example, the total volume of liquid added to the powder must be divided between the zinc and the NaOH solutions. Therefore, the concentration of the zinc solution was adjusted so that the total volume of zinc solution needed was reduced to 50% of the total liquid volume added to the peptide powder (step d). The remaining 50% of the total liquid volume added to the peptide powder was added as NaOH solution as detailed below. Preparations C3 and C4 were prepared using this method.

The pH adjusted semi-solid or gel compositions were prepared using a "push-pull" mixing method:

a) The desired amount of peptide was weighed into the barrel of a disposable syringe **S1** previously fitted with a special two-way hand valve **HV** (I.D.=0.5 mm) and tubing was placed inside the syringe Luer hole;
b) The syringe plunger was secured with a stainless steel rod **SR;**
c) **HV** in **S1** was connected to a vacuum source and **HV** was opened. After 10 min **HV** was closed;
d) The Zinc solution was accurately weighed into the barrel of a second disposable syringe **S2;**
e) **S2** was then connected to the free part of **HV;**
f) **HV** was opened and the solvent was pulled by the vacuum into the barrel containing the peptide powder **S1**;
g) **HV** was closed and the solvent syringe **S2** was removed, thus hydrating the peptide powder in **S1**;
h) **SR** was removed and the syringe plunger was slowly released;

i) The syringe plunger is moved (push and pull), without opening **HV,** so that the powder mass is fully soaked by solvent;

j) A two-way stainless connector **SC** (I.D.=1.0 mm) was placed in syringe **S2** with the tubing placed inside the syringe Luer hole, and its plunger was pushed to the end;

k) **HV** in **S1** was opened to vent the vacuum and then **HV** was removed. The syringe plunger was moved so that air in the syringe barrel was minimized;

l) **S1** and **S2** were connected by **SC** and the composition was kneaded from **S1** to **S2** through **SC;**

m) After homogenization, an aliquot of the mixed product was removed to determine the concentration of the peptide;

n) The remaining intermediate bulk product was accurately weighed and the amount of NaOH solution required to reach the desired pH was calculated;

o) The NaOH solution was accurately weighed into a third disposable syringe **S3**; and

p) The syringe plungers were slowly compressed to minimize the air in the syringe chambers. Both syringes were connected by **SC** and the composition was kneaded through **SC.**

Using the teachings of the instant application, one skilled in the art could vary the amounts of peptide and $ZnCl_2$ to achieve compositions other than the 25% described herein.

Table 1

| Ex. Peptide No. | *Peptide % | Solution | Peptide: Zn Ratio | **Peptide: Dose |
|---|---|---|---|---|
| C1 | 10 | $ZnCl_2$ 0.846 mg/ml | 5.4:1 | 1 mg |
| C2 | 5 | 0.40 mg $ZnCl_2$/ml | 5.4:1 | 1 mg |
| C3 | 10 | 50% $ZnCl_2$ 1.69 mg/ml, 50% NaOH 1 mg/ml | 5.4:1 | 1 mg |
| C4 | 10 | 50% $ZnCl_2$ 2.28 mg/ml, 50% NaOH 1 mg/ml | 4:1 | 1 mg |
| C5 | 5 | 80% $ZnCl_2$ 0.674 mg/ml, 20% NaOH 3.81 mg/ml | 4:1 | 1 mg |
| C6 | 2 | 80% $ZnCl_2$ 0.26 mg/ml, 20% NaOH 2.15 mg/ml | 5.4:1 | 1 mg |
| C7 | 10 | 80% $ZnCl_2$ 3.81 mg/ml, 20% NaOH 4.47 mg/ml | 1.5:1 | 1 mg |
| C8 | 10 | 80% $ZnAc_2.2H_2O$ 2.3 mg/ml, 20% NaOH 6.1 mg/ml | 4:1 | 1 mg |
| C9 | 2 | 80% $ZnCl_2$ 0.695 mg/ml, 20% NaOH 1.75 mg/ml | 1.5:1 | 1 mg |
| C10 | 2 | 80% $ZnAc_2.2H_2O$ 1.12 mg/ml, 20% NaOH 1.44 mg/m | 1.5:1 | 1 mg |
| C11 | 2 | 80% $ZnCl_2$ 0.695 mg/ml, 20% NaOH 1.75 mg/ml | 1.5:1 | 1 mg |
| C12 | 1 | 80% $ZnCl_2$ 0.384 mg/ml, 20% NaOH 0.875 mg/ml | 1.5:1 | 1 mg |
| C13 | 10 | 80% $ZnCl_2$ 3.85 mg/ml, 20% NaOH 4.47 mg/ml | 1.5:1 | 15 mg |

* Target value shown. Actual value was within 5% of target in all cases
** Target value shown. Actual values were within 10% of target in all cases

3.0 Determination of GLP-1 receptor affinity

A compound useful to practice the present invention can be tested for its ability to bind to the GLP-1 receptor using the following procedure.

Cell Culture:

RIN 5F rat insulinoma cells (ATCC-# CRL-2058, American Type Culture Collection, Manassas, VA), expressing the GLP-1 receptor, were cultured in Dulbecco's modified Eagle's medium (DMEM) containing 10% fetal calf serum, and maintained at about 37 °C in a humidifed atmosphere of 5% $CO_2$/95% air.

Radioligand Binding:

Membranes were prepared for radioligand binding studies by homogenization of the RIN cells in 20 ml of ice-cold 50 mM Tris-HCl with a Brinkman Polytron (Westbury, NY) (setting 6, 15 sec). The homogenates were washed twice by centrifugation (39,000 g / 10 min), and the final pellets were resuspended in 50 mM Tris-HCl, containing 2.5 mM $MgCl_2$, 0.1 mg/ml bacitracin (Sigma Chemical, St. Louis, MO), and 0.1% BSA. For assay, aliquots (0.4 ml) were incubated with 0.05 nM ($^{125}$I)GLP-1(7-36) (~2200 Ci/mmol, New England Nuclear, Boston, MA), with and without 0.05 ml of unlabeled competing test peptides. After a 100 min incubation (25 ˚C), the bound ($^{125}$I)GLP-1 (7-36) was separated from the free by rapid filtration through GF/C filters (Brandel, Gaithersburg, MD), which had been previously soaked in 0.5% polyethyleneimine. The filters were then washed three times with 5 ml aliquots of ice-cold 50 mM Tris-HCl, and the bound radioactivity trapped on the filters was counted by gamma spectrometry (Wallac LKB, Gaithersburg, MD). Specific binding was defined as the total ($^{125}$I)GLP-1(7-36) bound minus that bound in the presence of 1000 nM GLP1 (7-36) (Bachem, Torrence, CA).

4. Determination of solubility vs pH

4.1. Determination of Compound Solubility vs pH in Phosphate Buffered Saline (PBS)

A compound that may advantageously be used to practice the invention can be tested to determine its solubility in PBS at different pHs and temperatures using the following procedure.

A stock PBS buffered solution was made by dissolving one packet of premixed powder (SIGMA, Product No.: P-3813) in one liter of de-ionized water to yield 10 mM phosphate-buffered saline with 138 mM NaCl, 2.7 mM KCl, and a pH of 7.4. PBS buffers with different pH values were made by adjusting the pH of this stock solution with phosphoric acid and/or sodium hydroxide.

Two mg samples of a compound to be tested, e.g., 2 mg of the compound of Example 1, were weighed into glass vials. Into each vial was added a 50 μl aliquot of PBS buffer at a certain pH. The solution was vortexed, and if necessary sonicated, until clear. For each pH tested the total volume of buffer needed to dissolve 2 mg of the compound was recorded and the solubility was calculated.

Peptide solutions that are clear at room temperature (20-25˚ C) were placed in a refrigerator (4˚ C) overnight and the solubility of the peptide at 4˚ C was then examined.

4.2. Determination of compound solubility vs pH in saline

A compound that may advantageously be used to practice the invention can be tested to determine its solubility in saline at different pH values and temperatures using the following procedure.

A stock saline solution is prepared by dissolving 9 grams of NaCl in one liter of de-ionized water. Saline solutions with different pH values are made by adjusting the pH of this stock solution with HCl and/or NaOH.

Two mg samples of a compound to be tested, e.g., 2 mg of a compound of example 1, are weighed into glass vials. Into each vial is added a 50 μl aliquot of saline solution at a certain pH. The vial is vortexed and, if necessary, sonicated until clear. For each tested pH the total volume of saline needed to dissolve 2 mg of the compound is recorded and the solubility is calculated.

Solutions that are clear at room temperature (20-25˚ C) are placed in a refrigerator (4˚ C) overnight and the solubility at 4˚ C then examined.

4.3. Determination of compound solubility in saline at pH 7.0

Compounds that may advantageously be used to practice the invention can be tested to determine their solubility at room temperature in saline having pH = 7 using the following procedure.

Saline solution is prepared by dissolving 9 grams of NaCl in one liter of de-ionized water. A 2 mg sample of a compound to be tested, e.g., a compound of example 1, is weighed into a glass vial and 1 ml aliquots of saline are added, with vortexing and sonication, until clear. The total volume of saline used to dissolve 2 mg of peptide is recorded and the solubility at room temperature is calculated.

4.4. Determination of compound solubility in saline at various pH

Compounds that may advantageously be used to practice the invention can be tested to determine their solubility at room temperature in saline solutions having various pH values using the following procedure.

A stock saline solution is prepared by dissolving 9 grams of NaCl in one liter of de-ionized water. Saline solutions having various pH values are obtained by treating aliquots of this stock saline solution with HCl and NaOH.

A 2 mg sample of a compound to be tested, e.g., the compound of example 1, is weighed into a glass vial. Aliquots of 50 μl of a saline buffer at a certain pH are added. The solution is vortexed and sonicated until clear. The total volume of buffer used to dissolve 2 mg of peptide is recorded and the solubility is calculated.

## 5. Determination of aqueous solubility of compound vs zinc concentration

A compound that may advantageously be used to practice the invention can be tested to determine its solubility in pH 7 water at different zinc concentrations using the following procedure.

A stock zinc solution was prepared by dissolving $ZnCl_2$ in de-ionized water to a concentration of 100 mg/ml and adjusting the pH to 2.7 using HCl. Solutions having various $ZnCl_2$ concentrations ("Zn Test Solutions") were prepared by making appropriate dilutions of the stock solution.

One mg of a compound to be tested, e.g., 1 mg of the compound of Example 1, was dissolved in 250 $\mu$l of each Zn Test Solution to yield a solution having 4 mg/ml of the compound. The pH of this solution was then adjusted using 0.2 N NaOH until white precipitates were observed to form. The precipitation solution was centrifuged and the mother liquor analyzed using HPLC. The UV absorption area of test compound peak was measured and the concentration of the test compound in the mother liquor was determined via comparison to a calibration curve.

As a representative example of a compound that may be used to practice the invention, the compound of Example 1 was tested in the immediately foregoing assay and the following results were obtained (aqueous, pH 7.0, room temperature):

Table 2

| $ZnCl_2$ concentration ($\mu$g/ml) | Solubility (mg/ml) |
|---|---|
| 0 | 5.788 |
| 80 | 0.0770 |
| 500 | 0.0579 |
| 1000 | 0.0487 |
| 1500 | 0.0668 |
| 2500 | 0.1131 |

## 6. Determination of isoelectric point (pI) using IEF gels

Invitrogen's Novex IEF pH3-10 gels were used to measure the pI of GLP-1 peptides, e.g., the compound of Example 1. Peptidyl compounds to be tested were dissolved in water to a concentration of 0.5 mg/ml. For each such compound, 5 $\mu$l of the resulting solution was mixed with 5 $\mu$l of Novex® Sample Buffer 2X (comprised of 20 mM Arginine free base, 20 mM Lysine free base and 15% Glycerol) and the resulting 10 $\mu$l sample solution was loaded onto the gel along with a protein standard sample.

Running buffers were also obtained from Invitrogen and the gel is run according to manufacture's instructions, generally as follows: 100 V constant for 1 hour, followed by 200 V constant for 1 hour, followed by 500 V constant for 30 minutes.

The gel was then fixed in 12% TCA containing 3.5% sulfosalicylic acid for 30 minutes, and then stained for 2 hours with Colloidal Coomassie Blue according to the instructions found on the Novex® Colloidal Blue Kit thereafter, then destained in water overnight.

The gel was scanned and analyzed by the program Fragment Analysis 1.2. pI's of unknown peptides were calculated relative to the pI's of standard compounds having pI values of: 10.7, 9.5, 8.3, 8.0, 7.8, 7.4, 6.9, 6.0, 5.3, 5.2, 4.5, 4.2, and 3.5.

The measured pI of the compound of Example 1 was 7.60.

## 7. In vivo assays in rat

Compositions of the present invention can be tested to determine their ability to promote and enhanced effect *in vivo* using the following assays.

## 7.1. Experimental procedure:

The day prior to the experiment, adult male Sprague-Dawley rats (Taconic, Germantown, NY) that weighed approximately 300-350g were implanted with a right atrial jugular cannula under chlorohydrate anesthetic. The rats were then fasted for 18 hours prior to the injection of the appropriate test composition or vehicle control at time 0. The rats continued to be fasted throughout the entire experiment.

A 0.5 mg/ml $ZnCl_2$ solution was prepared by dilution of a solution of 100 mg/ml $ZnCl_2$ in an HCl solution having pH 2.7 water. 1 mg of the compound of formula (I) ((Aib$^{8,35}$)hGLP1(7-36)NH$_2$) was dissolved in 250 $\mu$l of this solution to yield a clear solution having 4 mg/ml of the compound and 0.5 mg/ml Zn at pH 4.

At time zero the rats were injected subcutaneously (sc) either with (a) the immediately forgoing solution of (Aib$^{8,35}$)hGLP-1(7-36)NH$_2$), or with vehicle control. In both cases the injection volume was very small (4-6 $\mu$L) and the dose of GLP-1 compound administered to the subject was 75 $\mu$g/kg. At the appropriate time after the sc injections a 500$\mu$l blood sample was withdrawn via the intravenous (iv) cannula and the rats were given an iv glucose challenge to test for the presence of enhanced insulin secretion. The times of the glucose challenge were 0.25, 1, 6, 12 and 24 hours post-compound injection. After the initial blood sample was withdrawn glucose (1 g/kg) was injected iv and flushed in with 500$\mu$l heparinized saline (10U/ml). Thereafter, 500$\mu$l blood samples were withdrawn at 2.5, 5, 10 and 20 minutes post-glucose injection. Each of these was immediately followed by an iv injection of 500$\mu$l heparinized saline (10U/ml) through the cannula. The blood samples were centrifuged, plasma was collected from each sample and the samples were stored at -20˚C until they were assayed for insulin content. The amount of insulin in each sample was determined using a rat insulin enzyme-linked immunosorbant assay (ELISA) kit (American Laboratory Products Co., Windham, NH).

7.1.1. Results:

A sustained insulin-enhancing activity was observed that was inducible by glucose injection over the full 24 hours of the experiment.

8. *In vivo* assays in dog

There are a number of *in vivo* assays known in the art which enable the skilled artisan to determine a composition's ability to promote extended release of active compound *in vivo.*

8.1. 1% Peptide Composition:

By way of example, an aqueous test formulation was prepared comprising 1% (w/w) of the compound of formula (I) in a buffered solution of ZnCl$_2$ (peptide:Zn ratio = 1.5:1.0).

A total of 6 male Beagle dogs, ages 42 - 78 months and 14 - 21 kg bodyweight were maintained with free access to water and once daily food (approx. 400 g of dry standard diet (SAFE 125). The dogs were fasted 18 hours before administration of test composition.

The test composition was administered by subcutaneous route in the interscapular area by. The volume of administration (approx. 20 microliters per animal) was made by 0.3 ml Terumo syringes with 0.33-12 mm (BS=30M2913). A theoretical dose of approximately 0.2mg peptide was thus achieved.

Blood samples were taken periodically, at approx. time = 0, 8, 15, 30, 45 min, and 1, 2, 4, 8, and 12 hours, and 1, 2, 3, 4, 5, and 6 days after administration. The blood was rapidly chilled after sampling until centrifugation, and the plasma decanted and rapidly frozen pending assay. Determination of peptide plasma concentration was made after off line solid phase extraction, followed by on-line phase extraction coupled to LC-MS/MS, and the data obtained managed by Analyst v1.2 software.

The composition demonstrated an extended release of the active peptide for at least 2 days.

8.2. 1% (Aib$^{8,35}$)hGLP1(7-36)NH$_2$) Solution:

Using substantially the same in vivo assay procedure as described in section 8.1, above, the following compositions were examined for their ability to release the subject peptide over an extended period of time. For each of the following four compositions the concentration of peptide was about 1% (wt/wt), the ratio of peptide to zinc was about 1.5:1, and the dose of peptide administered was approximately 1 mg.

Solution 8.2.A: (Aib$^{8,35}$)hGLP1(7-36)NH$_2$ in a solution containing (i) 90% ZnCl$_2$ (0.298 mg/ml) and (ii) 10% NaOH (0.975 mg/ml);

Solution 8.2.B: (Aib$^{8,35}$)hGLP1(7-36)NH$_2$ in a solution of ZnCl$_2$ (0.286 mg/ml)

Solution 8.2.C: Substantially similar to Solution 8.2.B, and buffered using AcOH/AcO$^-$

Solution 8.2.D: Substantially similar to Solution 8.2.A

The compositions provided for an extended release of (Aib$^{8,35}$)hGLP1(7-36)NH$_2$, as depicted in Figure 1.

8.3. 1% (ib$^{8,35}$)hGLP1(7-36)NH$_2$) Solution:

Using substantially the same *in vivo* assay procedure as described in section 8.1, above, the following composition was examined for its ability to release the subject peptide over an extended period of time. For the following composition the concentration of peptide was about 2% (wt/wt), the ratio of peptide to zinc was about 1.5:1, and the dose of peptide administered was approximately 1 mg.

Solution 8.3.: $(Aib^{8,35})hGLP1(7-36)NH_2$ in a solution containing (i) 80% $ZnCl_2$ (0.695 mg/ml) and (ii) 20% NaOH (1.75 mg/ml);

The composition provided for an extended release of $(Aib^{8,35})hGLP1(7-36)NH_2$, as depicted in Figure 5.

### 8.4. 10% Peptide Solutions:

Using substantially the same *in vivo* assay procedure as described in section 8.1, above, the following compositions were examined for their ability to release the subject peptide over an extended period of time. For each of the following four compositions the concentration of peptide was about 10% (wt/wt), the ratio of peptide to zinc was about 1.5:1, and the dose of peptide administered was approximately 15 mg.

Solution 8.4.A: $(Aib^{8,35})hGLP1(7-36)NH_2$ in a solution containing (i) 90% $ZnCl_2$ (3.367 mg/ml) and (ii) 10% NaOH (5.01 mg/ml);

Solution 8.4.B: $(Aib^{8,35})hGLP1(7-36)NH_2$ in a solution of $ZnCl_2$ (2.993 mg/ml)

Solution 8.4.C: Substantially similar to Solution 8.4.B, and buffered using $AcOH/AcO^-$

Solution 8.4.D: Substantially similar to Solution 8.4.A

The compositions provided for an extended release of $(Aib^{8,35})hGLP1(7-36)NH_2$, as depicted in Figure 2.

### 8.5. Semisolid Compositions:

Using substantially the same *in vivo* assay procedure as described in section 8.1, above, the following semisolid compositions were examined for their ability to release the subject peptide over an extended period of time. For composition 8.5.A., the concentration of the peptide was about 5%, while for compositions 8.5.B, 8.4.C, and 8.5.D., the concentration of peptide was about 10% (wt/wt). The ratio of peptide to zinc for compositions 8.5.A, 8.5.B, and 8.5.C was about 5.4:1, while for composition 8.5.D the ratio was about 4.0:1. For all four compositions the dose of peptide administered was approximately 1 mg.

Composition 8.5.A: $(Aib^{8,35})hGLP1(7-36)NH_2$ in a semisolid composition containing $ZnCl_2$ (0.40 mg/ml) in WFI.

Composition 8.5.B: Substantially similar to Composition 8.5.A., wherein the ZnCL2 concentration has been adjusted upward to maintain a peptide:Zn ratio of about 5.4:1.

Composition 8.5.C: $(Aib^{8,35})hGLP1(7-36)NH_2$ in a semisolid containing (i) 50% $ZnCl_2$ (1.69 mg/ml) and (ii) 50% NaOH (1mg/ml).

Composition 8.5.D: $(Aib^{8,35})hGLP1(7-36)NH_2$ in a semisolid containing (i) 50% $ZnCl_2$ (2.28 mg/ml) and (ii) 50% NaOH (1 mg/ml).

The compositions provided for an extended release of $(Aib^{8,35})hGLP1(7-36)NH_2$, as depicted in Figure 3.

### 8.6. Semisolid Compositions:

Using substantially the same *in vivo* assay procedure as described in section 8.1, above, the following semisolid composition was examined for its ability to release the subject peptide over an extended period of time. This composition was formulated using a 5.22 mg/ml $ZnCl_2$ solution, at pH = 2.0. Sufficient peptide was provided to result in a 25% peptide semisolid composition having a peptide to zinc ratio of about 4:1. The pH of the composition was adjusted as provided herein using 10 mg/ml NaOH. The dose of peptide administered was approximately 15 mg.

Composition 8.6 provided for an extended release of $(Aib^{8,35})hGLP1(7-36)NH_2$, as depicted in Figure 6.

### 8.7. Semisolid Compositions:

Using substantially the same in vivo assay procedure as described in section 8.1, above, the following semisolid composition was examined for its ability to release the subject peptide over an extended period of time. This composition was formulated using a 8.5 mg/ml $ZnCl_2$ solution, at pH = 2.0. Sufficient peptide was provided to result in a 23% peptide semisolid composition having a peptide to zinc ratio of about 1.5:1. The composition was formulated according to the process detailed in section 2.6, above. The dose of peptide administered was approximately 15 mg (corresponding to about 65 microliters of the composition).

Composition 8.6 provided for an extended release of $(Aib^{8,35})hGLP1(7-36)NH_2$, as depicted in Figure 7.

Further assays with various permutations of the disclosed formulation have likewise been subject to in vivo assay and have confirmed that compositions of the present invention provide a useful drug delivery platform for the compound of formula (I). Using the teachings of the instant application, one skilled in the art could vary the amounts of peptide, $ZnCl_2$ and pH to prepare compositions of the present invention as described herein.

Example 9

1. PK profile modulation by Acetate content in 10% peptide solutions.

This example discloses a pharmacokinetic study of $(Aib^{8,35})hGLP1(7-36)NH_2$ in male beagle dogs following by single subcutaneous administration of two extemporaneous compositions containing 10% $(Aib^{8,35})hGLP1(7-36)NH_2$ and zinc chloride [$(Aib^{8,35})hGLP1(7-36)NH_2$:Zn=1.5:1] at dose level of 15mg/dog.

The method to conduct the *in vivo* assay is the same as disclosed under paragraph 8.1.

This example illustrates PK profile modulation by acetate content in the pharmaceutical composition and thus the influence of the ratio [acetate/peptide] in the pharmaceutical composition on the pH.

The pH modulation is controlled by the way of modulation of acetate content a decreasing content of acetate shows an increasing effect on the pH.

A variation of acetate also shows an effect on the $C_{max}$. In general a decreasing content of acetate decreases the $C_{max}$ value.

An increased content of acetate shows an improvement on the solubility and the physical stability.

According to the formulation chosen, the improvement by the modulation of the ratio acetate/peptide on solubility or stability, is compensated by the modulation of the ratio peptide/Zn for instance on the $C_{max}$. This can be seen as a system with three possible variables to adjust stability, solubility, the pH or C max.

In this example the abbreviation SD means standard deviation. AUC means the Artemisinin areas under the plasma concentration-time curve.

The meaning of the abbreviation MRT is mean residence time (MRT) is a parameter for estimating the rate of bioavailability to compare MRT with tmax wich is the time of peak drug concentration. MRTt was calculated using data from zero time through the last sampling time.

In Table 3 are gathered the results of the 10% peptide composition batches having different [Acetate/Peptide] ratios and subcutaneous administration in beagle dogs. The peak drug in plasma concentration values, the ($C_{max}$) was 8.10 ng/ml (SD=1.80 ng/ml) for an [Acetate/Peptide] molar ratio of the [3.7:1], whereas the batch having a lower ratio [3.2:1] provided a $C_{max}$ value of 5.65 ng/ml (SD=2.61 ng/ml).

Table 3

| Formulation | | 10% 15mg | | 10% 15mg | |
| Ratio peptide/Zn | | 1.5:1 | | 1.5:1 | |
|---|---|---|---|---|---|
| Parameter | *Units* | MEAN (n=5) | S.D. | MEAN (n=4) | S.D. |
| Dose | $\mu g \cdot kg^{-1}$ | 857.7 | 131.0 | 694.8 | 46.5 |
| $t_{max}$ | *d* | 0.208 | 0.167 | 0.111 | 0.068 |
| $C_{max}$ | $ng \cdot ml^{-1}$ | 8.10 | 1.80 | 5.65 | 2.61 |
| $t_{1/2\ app}$ | *d* | 3.32 | 0.66 | 6.77 | 2.04 |
| $AUC_t$ | $ng \cdot ml^{-1} \cdot d$ | 53.5 | 14.3 | 38.2 | 9.2 |
| AUC | $ng \cdot ml^{-1} \cdot d$ | 55.4 | 15.7 | 41.6 | 8.9 |
| $AUC_{extrap.}$ | % | 2.99 | 1.83 | 8.44 | 5.00 |
| $MRT_t$ | *d* | 9.31 | 2.25 | 7.48 | 1.39 |
| MRT | *d* | 9.96 | 2.60 | 9.85 | 2.54 |
| [Acetate: Peptide] | | 3.7:1 | | 3.2:1 | |

10. GLP-1 peptide salt/divalent metal formulations

10.1. Methods

$(Aib^{8,35})hGLP-1(7-36)NH_2$ 1 mg/ml water and PBS solutions were prepared and the pH was adjusted to 7.0. 10 mg/ml stock solutions of $CaCl_2$, $CuCl_2$, $MgCl_2$, and $ZnCl_2$ in water were prepared. The pH of $CaCl_2$, $MgCl_2$, and $ZnCl_2$ solutions was adjusted to 7.0. The pH of $CuCl_2$ solution could not be basified because Cu precipitated out. Therefore, $CuCl_2$ solution of pH 4.4 was used.

4 $\mu$L metal ion water or PBS solutions were added to 200 $\mu$L $(Aib^{8,35})hGLP-1(7-36)NH_2$ 1 mg/ml solution to make a final metal ion concentration of 200 $\mu$g/ml. The resulting solution was mixed and checked for precipitation. If precipitation formed, the suspension was centrifuged. The (Aib[8,35])hGLP-1(7-36)NH$_2$ concentration in the supernatant was determined by HPLC.

10.2. Results

Table 4. Solubility of (Aib[8,35])hGLP-1(7-36)NH$_2$ in the presence of divalent metal ions

|  | Water solution, mg/ml | PBS solution, mg/ml |
|---|---|---|
| CaCl$_2$ | >1 (pH 7.1) | >1 (pH 6.8) |
| CuCl$_2$ | 0.058 (pH 7.1) | 0.039 (pH 6.8) |
| MgCl$_2$ | >1 (pH 7.2) | >1 (pH 6.9) |
| ZnCl$_2$ | 0.108 (pH 6.9) | 0.056 (pH 6.8) |

10.3. Pharmacokinetic studies of (Aib[8,35])hGLP-1(7-36)NH$_2$/divalent metal pH 5.5 clear solution formulations

Three different formulations of (Aib[8,35])hGLP-1(7-36)NH$_2$ were prepared by using the following procedures:

(1) (Aib[8,35])hGLP-1(7-36)NH$_2$ HCl salt with CuCl$_2$

(2) (Aib[8,35])hGLP-1(7-36)NH$_2$ HCl salt with ZnCl$_2$

(3) (Aib[8,35])hGLP-1(7-36)NH$_2$ acetate salt with ZnCl$_2$

A TFA salt of a GLP-1 analog (the TFA salt results from the purification of the peptide by using preparative HPLC, eluting with TFA containing buffer solutions) can be converted into another salt, such as an acetate salt by dissolving the peptide in a small amount of 0.25 N acetic acid aqueous solution. The resulting solution is applied to a semi-prep HPLC column (Zorbax, 300 SB, C-8). The column is eluted with (1) 0.1 N ammonium acetate aqueous solution for 0.5 hrs., (2) 0.25N acetic acid aqueous solution for 0.5 hrs. and (3) a linear gradient (20% to 100% of solution B over 30 min.) at a flow rate of 4 ml/min (solution A is 0.25N acetic acid aqueous solution; solution B is 0.25N acetic acid in acetonitrile/water, 80:20). The fractions containing the peptide are collected and lyophilized to dryness.

(Aib[8,35])hGLP-1(7-36)NH$_2$ HCl salt was prepared by a lyophilization procedure. 20 mg (Aib[8,35])hGLP-1(7-36) NH$_2$ acetate was dissolved in 4 ml 20 mM HCl aqueous solution and incubated at room temperature for 10 min. The sample was frozen and lyophilized overnight. Lyophilization was performed for another two times and the chloride content of the final product was determined. The determined chloride content was 5.38%.

(1) (Aib[8,35])hGLP-1(7-36)NH$_2$ HCl salt with CuCl$_2$:

(Aib[8,35])hGLP-1(7-36)NH$_2$ HCl 5.3 mg (peptide content is 95%) was dissolved in 50 μL 20 mM CuCl$_2$ aqueous solution. The pH was adjusted with approximately 2 μL 1 N NaOH to about 5.5. The molar ratio of (Aib[8,35])hGLP-1(7-36) NH$_2$/CuCl$_2$ was 1.5:1. The peptide concentration was 10% (30 mM) in water (w/w) with a pH of approximately 5.5.

(2) (Aib[8,35])hGLP-1(7-36)NH$_2$ HCl salt with ZnCl$_2$:

(Aib[8,35])hGLP-1(7-36)NH$_2$ HCl 5.3 mg (peptide content is 95%) was dissolved in 50 μL 20 mM ZnCl$_2$ aqueous solution. The pH was adjusted with approximately 2 μl of 1 N NaOH to about 5.5. The molar ratio of (Aib[8,35])hGLP-1 (7-36)NH$_2$/ZnCl$_2$ was 1.5:1. The peptide concentration was 10% (30 mM) in water (w/w) with a pH of approximately 5.5.

(3) (Aib[8,35])hGLP-1(7-36)NH$_2$ acetate salt with ZnCl$_2$:

(Aib[8,35])hGLP-1(7-36)NH$_2$ acetate 5.5 mg (peptide content is 92%) was dissolved in 50 μL 20 mM ZnCl$_2$ aqueous solution. The resulting solution was lyophilized overnight and redissolved in 50 μL water. The pH was adjusted with approximately 1 μL of 1 N NaOH to about 5.5. The molar ratio of (Aib[8,35])hGLP-1(7-36)NH$_2$/ZnCl$_2$ was 1.5:1. The peptide concentration was 10% (30 mM) in water (w/w) with a pH of approximately 5.5.

10.4. Dosing and blood sample collection

Rats were dosed at 0.3 mg/rat (3 $\mu$L of 10% solution) subcutaneously with these three formulations of (Aib[8,35]) hGLP-1(7-36)NH$_2$. Blood samples were collected at 5, 10, 15, 30 min, 1, 2, 4, 8 hours, and 1, 2, 3, 4, 7, 10 days. Plasma was collected from the blood by centrifugation and stored at -80°C. The tissue at the injection site was also collected, homogenized in 5x methanol, and stored at - 80°C.

Two rats were used for the 5, 10, 15, 30 min, and 1, 2, 4, 8 hours data points. One rat was used for 1, 2, 3, 4, 7, 10 days data points.

10.5. LC-MS/MS sample preparation

Plasma (200 $\mu$L) was acidified with 10 $\mu\lambda$L formic acid and precipitated with 600 $\mu$l acetonitrile. The supernatant was collected by centrifugation and concentrated to dryness under vacuum. The residues were dissolved in 150 $\mu$l 30% acetonitrile in water and centrifuged. 50 $\mu$l of the supernatant was injected for LC-MS/MS analysis.

Tissue methanol extract (10 $\mu$L) was diluted to 1 ml 30% acetonitrile in water and 50 $\mu$l was injected for LC-MS/MS analysis.

10.6. LC-MS/MS analysis

LC-MS/MS analysis was done with an API4000 mass spectrometer system equipped with a Turbo Ionspray probe. The MRM mode of molecular ion detection was used with the ion pair of 668.9 and 136.1.

HPLC separation was performed with a Luna C8(2) 2x30 mm 3$\mu$ column run from 10% B to 90% B in 10 minutes at a flow rate of 0.30 ml/minute. Buffer A is 1% formic acid in water and buffer B is 1% formic acid in acetonitrile.

LOQ was 0.5 ng/ml.

10.7. Results and summary

The plasma concentrations of the peptide were calculated with its standard calibration plot. 0.06 mg/ml (Aib[8,35]) hGLP-1(7-36)NH$_2$ (0.3 mg/rat in 5 ml methanol extract) was used as the 100% to calculate the percentages left at the injection sites.

Table 5. (Aib[8,35])hGLP-1(7-36)NH$_2$ plasma concentrations

| Time h | Plasma concentration (ng/ml) of (Aib[8,35])hGLP-1(7-36)NH$_2$ HCl and CuCl$_2$ dose | Plasma concentration (ng/ml) of (Aib[8,35])hGLP-1(7-36)NH$_2$ HCl and ZnCl$_2$ dose | Plasma concentration (ng/ml) of (Aib[8,35])hGLP-1(7-36)NH$_2$ acetate and ZnCl$_2$ dose |
|---|---|---|---|
| 0.083 | 4.76 | 5.06±3.85 | 25.9±14.57 |
| 0.17 | 3.18 | 13.04±12.81 | 16.35±5.02 |
| 0.25 | 3.44 | 13.65±8.14 | 32.2 |
| 0.5 | 7.95±5.3 | 13.86±11.8 | 19.5±3.68 |
| 1 | 11.8 | 12.4±10.61 | 11.5 |
| 2 | 11.4±1.27 | 12.9±0.35 | 8.64 |
| 4 | 5.9±5.2 | 6.39±4.62 | 5.48 |
| 8 | 0.9±0.37 | 0.72 | 6.41 |
| 24 | 1.35 | 1.08 | 0.94 |
| 48 | 0.68 | 1.21 | |
| 72 | 0.66 | 0.47 | 0.77 |
| 96 | 0.15 | 1.35 | 0.33 |
| 168 | 0.17 | 0.74 | 0.82 |
| 240 | 0.35 | 0.6 | 1.09 |

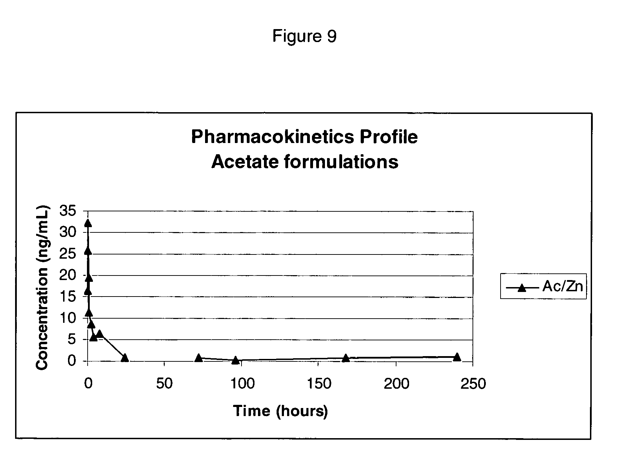

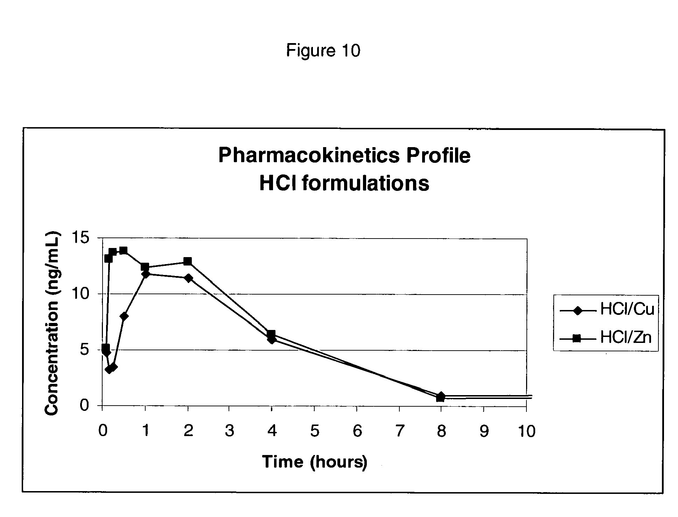

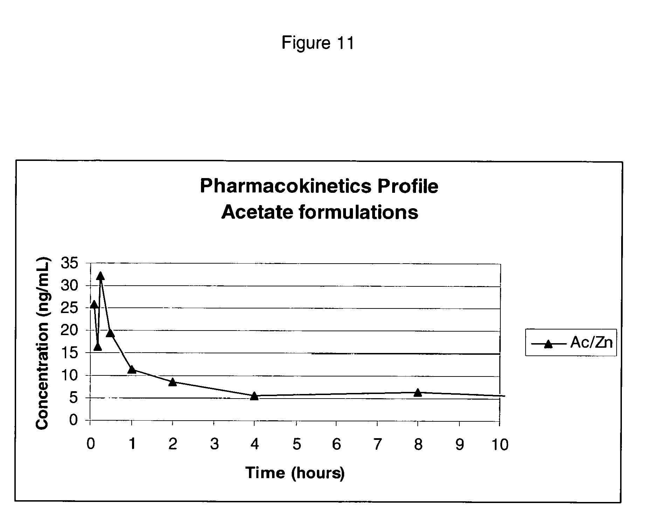

A full time course plot of the pharmacokinetics profile of the HCl salt formulations of $(Aib^{8,35})$hGLP-1(7-36)$NH_2$ is shown in Figure 8. An early time course plot of the pharmacokinetics profile of the HCl salt formulations of $(Aib^{8,35})$ hGLP-1(7-36)$NH_2$ is shown in Figure 9. A full time course plot of the pharmacokinetics profile of the acetate salt formulation of $(Aib^{8,35})$hGLP-1(7-36)$NH_2$ is shown in Figure 10. An early time course plot of the pharmacokinetics profile of the acetate salt formulation of $(Aib^{8,35})$hGLP-1(7-36)$NH_2$ is shown in Figure 11.

Table 6. Estimated percentages of $(Aib^{8,35})$hGLP-1(7-36)$NH_2$ left at the injection sites

| Time days | Estimated percentage (%) left at injection site of $(Aib^{8,35})$ hGLP-1(7-36)$NH_2$ HCl and $CuCl_2$ dose | Estimated percentage (%) left at injection site of $(Aib^{8,35})$ hGLP-1(7-36)$NH_2$ HCl and $ZnCl_2$ dose | Estimated percentage (%) left at injection site of $(Aib^{8,35})$ hGLP-1(7-36)$NH_2$ acetate and $ZnCl_2$ dose |
|---|---|---|---|
| 1 | 1.58 | 10.59 | 6.96 |
| 2 | 24.88 | 26.94 | 9.97 |
| 3 | 12 | 21.87 | 11.6 |
| 4 | 0.14 | 0.04 | 0.23 |
| 7 | 0.47 | 0.06 | 0.03 |
| 10 | 0.01 | 0.02 | 0.01 |

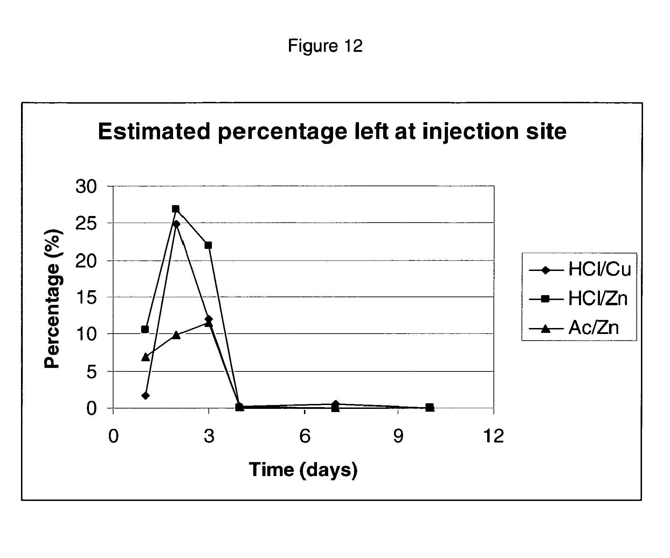

Tissue accumulation profile of $(Aib^{8,35})$hGLP-1(7-36)$NH_2$ at the injection site is further shown in Figure 12.

Table 7. PK parameters

| | Plasma concentration (ng/ml) of $(Aib^{8,35})$hGLP-1(7-36)$NH_2$ HCl and $CuCl_2$ dose | Plasma concentration (ng/ml) of $(Aib^{8,35})$hGLP-1(7-36)$NH_2$ HCl and $ZnCl_2$ dose | Plasma concentration (ng/ml) of $(Aib^{8,35})$hGLP-1(7-36)$NH_2$ acetate and $ZnCl_2$ dose |
|---|---|---|---|
| $T_{max}$, h | 1 | 0.5 | 0.25 |
| $C_{max}$, ng/ml | 11.8 | 13.8 | 32.2 |
| AUC ng-hr/ml | 204 | 514 | 394 |

The results indicate that salt forms of GLP-1 analogs, particularly in combination with a divalent metal salts, provide for acceptable sustained release formulations with reduced initial plasma concentrations, which may reduce or eliminate unwanted side-effects.

The data indicate that strong acid salts, for example, HCl salts of the GLP-1 analog, show a further reduction in initial plasma concentrations. Without being bound to this theory, it is believed that the superior reduction in initial plasma concentrations of the HCl salts of GLP-1 analogs relate to the neutralization process *in vivo.* In compositions (1) and (2) above at pH 5.5, 100% of the acid is in the chloride form and there is no free acid. Accordingly, after the subcutaneous injection the body fluid is able to neutralize the solution more quickly thereby precipitating the solution more rapidly. These decrease in neutralization time leads to a smaller, less pronounced, initial plasma concentration or spike.

Example 11: Preparation of liquid compositions with 10% peptide analogue $(Aib^{8,35})$hGLP-1(7-36)$NH_2$, acetic acid and zinc chloride

| Component | Example 11.1 Amount (mg/vial) | Example 11.2 Amount (mg/syringe) | Example 11.3 Amount (mg/syringe) |
|---|---|---|---|
| peptide analogue | 30 | 11 | 21 |
| acetic acid, glacial | 3.2 mol of acid per peptide mol | 3.2 mol of acid per peptide mol | 3.2 mol of acid per peptide mol |
| Zinc chloride | 0.816 | 0.299 | 0.571 |
| WFI | q.s. ad 300 | q.s. ad 110 | q.s. ad 210 |
| Final pH | 4.5 ±0.1 | 4.5 ±0.1 | 4.5 ±0.1 |

The syringe contained an overfill of 10 μl. In Example 11.1 the vial contains 0.3 ml of a 10% peptide analogue solution. In Example 11.2, the syringe contains 0.11 ml of a 10% peptide analogue solution. In Example 11.3 the syringe contains 0.21 ml of a 10 % peptide analogue solution. The quantity of peptide analogue was adjusted based on the assay of the drug substance. Sufficient acetic acid was added to provide a ratio of acetate to peptide of 3.2:1 mol equivalent. Sufficient zinc chloride was added to provide a ratio of peptide analogue to zinc chloride of 1.5:1 mol equivalent. Sufficient water for injection was added to provide a weight percentage of peptide analogue in the solution of 10%.

Method:

- Acetic acid was added in about 90% of WFI.
- Peptide analogue was added.
- Resulting composition was stirred until complete dissolution.
- Zinc chloride was added.
- The requisite amount of WFI was added.
- The resulting composition was stirred until complete dissolution.
- The dissolved solution underwent double sterilising filtration on 0.22 μm (MILLIPAK® 20).
- Filtered solutions were filled in vials 0.3 ml/vial = 0.310 g/vial (density=1.033) or in syringe including 10 μl as dead volume.

The publications cited above are incorporated herein by reference. Additional embodiments of the present invention will be apparent from the foregoing disclosure and are intended to be encompassed by the invention as described fully herein and defined in the following claims.

**Claims**

1.  A sustained release liquid pharmaceutical composition comprising:

    • a liquid;
    • a peptide analogue according to formula (I):

$$[Aib^{8,35}]hGLP\text{-}1(7\text{-}36)NH_2;$$

    • a divalent metal and/or divalent metal salt; and
    • an acetate salt and/or acetic acid.

2.  The pharmaceutical composition according to claim 1 wherein a portion of the peptide analogue and a portion of the acetate salt are present as a salt form of the peptide analogue.

3.  The pharmaceutical composition according to claim 1 or 2 wherein the final pH of the pharmaceutical composition is within the range of 3.5 to 6, preferably within the range of 4 to 5.

4.  The pharmaceutical composition according to claim 3 wherein the final pH of the pharmaceutical composition is 4.5±0.1

5.  The pharmaceutical composition according to any one of the preceding claims wherein the molar ratio range of the acetate salt and/or acetic acid to the peptide analogue is approximately 0.5:1 to approximately 10:1, preferably approximately 1:1 to approximately 6:1, more preferably approximately 3.2:1.

6.  The pharmaceutical composition according to any one of the preceding claims wherein the divalent metal and/or divalent metal salt is zinc chloride.

7.  The pharmaceutical composition according to claim 6 wherein the molar ratio range of the peptide analogue to zinc is approximately 6:1 to approximately 1:1, preferably approximately 1.5:1.

8.  The pharmaceutical composition according to any one of the preceding claims wherein the concentration of the peptide analogue is about 10 % by weight.

9. The pharmaceutical composition according to claim 8 wherein the composition is formulated such that the compound according to formula (I) is released within the subject for at least approximately 1 week, preferably 2 weeks.

10. The pharmaceutical composition according to any one of the previous claims wherein the liquid is selected from sterile water or a sterile water comprising an isotonic agent such as NaCl.

11. The pharmaceutical composition according to any one of the preceding claims, wherein the composition is stable at a temperature of 5 °C for a period of at least one year.

12. A container comprising the pharmaceutical composition according to any one of the preceding claims.

13. The container of claim 12 being a prefilled syringe.

14. A method for preparing the pharmaceutical composition of any one of the preceding claims, comprising the steps of:

A. combining the liquid, acetate salt and/or acetic acid and the peptide analogue; and
B. adding and dissolving the divalent metal and/or divalent metal salt.

15. The method of claim 14 comprising the further steps of:

C. sterile filtrating the composition resulting from Step B; and
D. filling a container with the composition.

Figure 1

Figure 2

Figure 3

Figure 4

Figure 5

Figure 6

Figure 7

Figure 8

Figure 9

Figure 10

Pharmacokinetics Profile
HCl formulations

Figure 11

Pharmacokinetics Profile
Acetate formulations

Figure 12

**Europäisches Patentamt**
**European Patent Office**
**Office européen des brevets**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/082656 A (SOD CONSEILS RECH APPLIC [FR]; DONG ZHENG XIN [US]; CHERIF-CHEIKH ROLA) 10 July 2008 (2008-07-10)<br>* page 6, line 8 - line 16 *<br>* page 5, line 25 - line 30 *<br>* page 7, line 3 - line 16 *<br>* page 11, line 6 - line 13 *<br>* page 14, line 33 - line 36 *<br>* page 38, line 10 - line 18 *<br>* page 35, line 11 - line 12 *<br>* page 36, line 6 - line 7 *<br>* page 41, line 23 - line 29; tables 5-7 *<br>----- | 1-15 | INV.<br>A61K38/26<br>A61K9/08 |
| X | WO 2007/005738 A (SOD CONSEILS RECH APPLIC [FR]; DONG ZHENG XIN [US]; CHERIF-CHEIKH ROLA) 11 January 2007 (2007-01-11)<br>* page 5, line 16 - line 21 *<br>* page 5, line 3 - line 5 *<br>* page 7, line 15 - line 17 *<br>* page 11, line 10 - line 26 *<br>* page 27, line 22 - line 23 *<br>* page 28, line 12 - line 13 *<br>solutions 8.2C and 8.4.C on pages 27 and 28<br>----- | 1-15 | |
| X | US 2007/004616 A1 (CHERIF-CHEIKH ROLAND [ES] ET AL) 4 January 2007 (2007-01-04)<br>* paragraphs [0010] - [0013], [0015], [0016], [0057] *<br>----- | 1-15 | |

**TECHNICAL FIELDS SEARCHED (IPC)**

A61K

The present search report has been drawn up for all claims

| Place of search | Date of completion of the search | Examiner |
|---|---|---|
| Munich | 3 July 2009 | Pilling, Stephen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 216 042 A1

## ANNEX TO THE EUROPEAN SEARCH REPORT
## ON EUROPEAN PATENT APPLICATION NO.

EP 09 29 0090

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2009

| Patent document cited in search report | | Publication date | Patent family member(s) | | Publication date |
|---|---|---|---|---|---|
| WO 2008082656 | A | 10-07-2008 | NONE | | |
| WO 2007005738 | A | 11-01-2007 | AR | 055346 A1 | 22-08-2007 |
| | | | AU | 2006265814 A1 | 11-01-2007 |
| | | | CA | 2617859 A1 | 11-01-2007 |
| | | | CN | 101258163 A | 03-09-2008 |
| | | | EP | 1904525 A2 | 02-04-2008 |
| | | | KR | 20080028981 A | 02-04-2008 |
| US 2007004616 | A1 | 04-01-2007 | NONE | | |

EPO FORM P0459

For more details about this annex : see Official Journal of the European Patent Office, No. 12/82

39

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0007617 A
- US 20040266670 A
- US 20050009742 A
- US 20020115605 A
- WO 03033697 A
- US 20040053819 A
- US 20030220251 A
- US 20030224983 A
- US 20040018975 A
- WO 9819698 A
- US 20030232754 A
- WO 9964060 A
- US 20040162241 A
- WO 9808531 A
- WO 0016797 A
- US 20040002454 A
- US 6006753 A
- US 20050096276 A
- US 20050037958 A

- US 20040266678 A
- US 20040029784 A
- US 20040235726 A
- US 20040209814 A
- US 20040209803 A
- US 20040097411 A
- US 20030216292 A
- US 20030199445 A
- US 20030036504 A
- US 20030143183 A
- US 20020147131 A
- US 20020045636 A
- WO 0157084 A
- EP 0619322 A2
- US 6566490 B
- US 6555521 B
- WO 0034331 A
- EP 9909660 W

**Non-patent literature cited in the description**

- **Varndell, J.M. et al.** *J. Histochem Cytochem,* 1985, vol. 33, 1080-6
- **Kreymann, B. et al.** *Lancet,* 1987, vol. 2, 1300-4
- **Wettergren A. et al.** *Dig Dis Sci,* 1993, vol. 38, 665-73
- **D'Alessio, D.A. et al.** *J. Clin Invest,* 1994, vol. 93, 2293-6
- **Gutniak, M.K. et al.** *Diabetes Care,* 1994, vol. 17, 1039-44
- **Williams, B. et al.** *J. Clin Endo Metab,* 1996, vol. 81, 327-32
- **Holz, G.G. 4 et al.** *Nature,* 1993, vol. 361, 362-5
- **Orskov, C.** *Diabetologia,* 1992, vol. 35, 701-711
- Potential of GLP-1 in diabetes management in Glucagon III. **Holst, J.J. et al.** Handbook of Experimental Pharmacology. Springer Verlag, 1996, 311-326
- **Kreymann, B. et al.** *Lancet,* 1987, vol. ii, 1300-1304
- **Weir, G.C. et al.** *Diabetes,* 1989, vol. 38, 338-342
- **Gutniak, M.** *N. Engl J Med,* 1992, vol. 226, 1316-1322

- **Nathan, D.M. et al.** *Diabetes Care,* 1992, vol. 15, 270-276
- **Nauck, M.A. et al.** *Diabetologia,* 1993, vol. 36, 741-744
- **Creutzfeldt, W.O. et al.** *Diabetes Care,* 1996, vol. 19, 580-586
- **Deacon, C.F. et al.** *Diabetes,* 1995, vol. 44, 1126-1131
- **Kim ; Haren.** *Pharma. Res.,* 1995, vol. 12 (11
- **Pridal.** *International Journal of Pharmaceutics,* 1996, vol. 136, 53-59
- **Choi et al.** *Pharm. Research,* 2004, vol. 21 (5
- **Ritzel et al.** *Diabetologia,* 1995, vol. 38, 720-725
- **Gutniak et al.** *Diabetes Care,* 1994, vol. 17 (9), 1039-1044
- **Deacon et al.** *Diabetes,* 1995, vol. 44, 1126-1131
- **Mojsov, S.** *Int. J. Peptide Protein Res,* 1992, vol. 40, 333-342
- **Todd, J.F. et al.** *Clinical Science,* 1998, vol. 95, 325-329
- **Todd, J.F. et al.** *European Journal of Clinical Investigation,* 1997, vol. 27, 533-536
- **Stewart, J.M. et al.** Solid Phase Synthesis. Pierce Chemical Co, 1984

- **Schnolzer et al.** *Int. J. Peptide Protein Res.,* 1992, vol. 40, 180